(19) Europäisches Patentamt
European Patent Office
Office européen des brevets

(11) **EP 3 435 979 B1**

(12) **FASCICULE DE BREVET EUROPEEN**

(45) Date de publication et mention
de la délivrance du brevet:
**14.06.2023   Bulletin 2023/24**

(21) Numéro de dépôt: **17714305.4**

(22) Date de dépôt: **29.03.2017**

(51) Classification Internationale des Brevets (IPC):
*A61K 9/08* (2006.01)   *A61K 33/24* (2019.01)
*A61K 33/40* (2006.01)   *C11D 3/48* (2006.01)
*A61P 31/22* (2006.01)   *A61P 31/12* (2006.01)

(52) Classification Coopérative des Brevets (CPC):
(C-Sets disponibles)
**A61K 33/24; A61K 9/0014; A61K 9/0034;
A61K 9/0048; A61K 9/006; A61K 9/08;
A61K 33/242; A61K 33/244; A61K 33/40;
A61K 47/08; A61K 47/183; A61P 31/22**   (Cont.)

(86) Numéro de dépôt international:
**PCT/IB2017/051797**

(87) Numéro de publication internationale:
**WO 2017/168344 (05.10.2017 Gazette 2017/40)**

(54) **COMPOSITION, NOTAMMENT COMPOSITION PHARMACEUTIQUE PRÉVENTIVE ET CURATIVE, À BASE DE PEROXOMÉTALLATE**

AUS PEROXOMETALLAT HERGESTELLTE ZUSAMMENSETZUNG, INSBESONDERE EINE VORBEUGENDE UND HEILENDE PHARMAZEUTISCHE ZUSAMMENSETZUNG

COMPOSITION, IN PARTICULAR A PREVENTIVE AND CURATIVE PHARMACEUTICAL COMPOSITION, MADE FROM PEROXOMETALLATE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **29.03.2016   FR 1652697**

(43) Date de publication de la demande:
**06.02.2019   Bulletin 2019/06**

(73) Titulaire: **Oxymo Technologies Inc.
Longueuil, Québec J4G 2V8 (CA)**

(72) Inventeurs:
• **CHRETIEN, Denis Olivier
92250 La Garenne Colombes (FR)**
• **WILMOTTE, Rémi
51140 Chalons-sur-Vesles (FR)**
• **LORENZO, Frédéric
77240 Seine-Port (FR)**

(74) Mandataire: **Lavoix
2, place d'Estienne d'Orves
75441 Paris Cedex 09 (FR)**

(56) Documents cités:
**WO-A1-2005/010774     WO-A2-2010/004161
US-A1- 2007 059 255**

EP 3 435 979 B1

(52) Classification Coopérative des Brevets (CPC):
(Cont.)

C-Sets
**A61K 33/24, A61K 2300/00;**
**A61K 33/40, A61K 2300/00**

## Description

La présente invention concerne un mélange actif dans un traitement thérapeutique, ledit mélange comprenant un peroxométallate, comme par exemple un peroxomolybdate et/ou, comme par exemple un peroxolanthanate.

En particulier, la présente invention concerne un mélange actif pour le traitement préventif et curatif des infections à *Herpesviridae* et, en particulier, les infections à *Herpes simplex* 1 (HSV-1 ou HHV-1) et à *Herpes simplex* 2 (HSV-2 ou HHV-2). La présente invention concerne aussi des compositions pharmaceutiques comprenant un tel mélange actif, des méthodes de préparation ainsi que ses utilisations, en particulier dans le cadre d'un traitement thérapeutique.

## État de la technique

Parmi les pathologies infectieuses, celles dues aux virus, outre leurs gravités variables selon leurs types, pouvant aller jusqu'au décès du patient, et avec un taux de morbidité très inégal selon les populations et les époques, posent un problème thérapeutique majeur. Cet agent causal nécessite un hôte cellulaire dont il utilise le métabolisme et les constituants pour se multiplier, d'où la difficulté d'éradication par traitement respectueux du tissu sain par rapport à celui infecté, voire un traitement préventif.

Parmi les infections virales pouvant affecter l'homme, celles à *Herpesviridae* sont courantes, très contagieuses et endémiques. Cette famille de virus à ADN est constituée de huit types de virus. Parmi eux, HHV-1 ou HSV-1 et HHV-2 ou HSV-2 sont responsables des herpès orofaciaux-labiaux et vaginaux-anaux.

Tous les herpès-virus, partagent des propriétés structurales protéiques et génomiques, cliniques comme la latence, la récurrence ou l'activation lytique. Comme pour toutes les infections virales, les moyens thérapeutiques sont complexes et souvent limités. L'invention s'attache à résoudre le problème technique consistant en la fourniture d'une composition pharmaceutique permettant de lutter contre une infection virale, en particulier d'une infection virale impliquant les *Herpesviridae* et en particulier HSV-1 et HSV-2.

Les infections à HSV-1 et HSV-2 sont très contagieuses (principalement par contact) et endémiques (Organisation Mondiale de la Santé (OMS), janvier 2016, "Le virus de l'herpès", aide mémoire N°400, http://www.who.int/mediacentre/factsheets/fs400/fr/#). Le réservoir est humain, sans zoonose ni vecteur. La prévalence des infections à HSV-1 responsables de l'herpès orofacial-labial, de certaines formes d'herpès génital et du panaris herpétique, est variable selon les continents, les méthodes et les possibilités de diagnostics. Elle est estimée, en moyenne, de 84 à 99% en Afrique, de 50 à 100% en Asie, de 65 à 98% en Europe et de 57 à 68% en Amérique du Nord. La prévalence des infections à HSV-2, responsable de l'herpès génital et anal, est variable selon le sexe, le profil sociologique et ethnique, l'âge et le continent. Par exemple (d'après : Centers for Disease Control and Prévention (CDC), 2010, MMW R Morb Mortal Wkly Rep., 59(15):456-459), la séroprévalence aux Etats-Unis pour la tranche d'âge de 40 à 49 ans est estimée à 32% chez les femmes et à 20% chez les hommes.

En considérant le taux de morbidité, les infections à HSV-1 et HSV-2 ont un impact mondial certain. En effet, il n'existe aucun traitement curatif totalement efficace en termes d'éradications, que l'administration d'antiviraux soit *per os* ou par voie parentérale, que ce soit d'un analogue de pyrophosphate (le foscarnet, par exemple), d'analogues de nucléosides non activables ou activables *in situ* (comme la fiacitabine, la vidarabine, l'aciclovir et sa prodrogue le valaciclovir, par exemple) ou d'un inhibiteur du complexe enzymatique hélicase-primase (le pritelivir). Ils aident à réduire la gravité et la fréquence des symptômes mais ne peuvent pas éradiquer l'infection. Le plus souvent, en conséquence de la faible solubilité de ces molécules et en particulier celles de la famille des "ciclovir" (1.3 mg/mL dans l'eau à 25°C pour l'aciclovir) ainsi que de leur absorption intestinale limitée (de l'ordre de 20% pour l'aciclovir *per os*), des doses conséquentes doivent être administrées aux patients.

Contrairement aux topiques ophtalmiques, les substances anti-HSV utilisées en dermatologie (comme le ciclofovir, l'ibacitabine, l'aciclovir, par exemple) ont une efficacité clinique qui reste modeste et inconstante.

De plus, des formes de résistances acquises du virus de l'*Herpes simplex* aux traitements classiques *per os* ou par voie parentérale sont décrites en émergence. Elles sont le plus souvent reliées à des mutations du génome viral (Bacon T.H. et al, 2003, Clin. Microbiol. Rev., 16(1), 114-128; Biswas S. et al, 2008, Antiviral Res., 80(1), 81-85).

Outre les volets de contagiosité, d'algie et un aspect inesthétique récurrent du patient lors de la réactivation virale, ces infections peuvent se compliquer par des pathologies graves et complexes bien que demeurant assez rares, comme l'encéphalite herpétique ou la kératite. La contamination néonatale maternelle peut être mortelle à 60% en absence de traitement. Les infections herpétiques et leur développement peuvent être associés à d'autres pathologies du fait d'immunodépression (induite ou pathologique) ou d'immunosuppression du patient comme par exemple, dans le cas de certains cancers ou après une greffe. HSV-2 fait partie des infections les plus courantes (de 60 à 90%) parmi les personnes porteuses du VIH. Le risque de contracter une nouvelle infection à VIH est multiplié par trois en présence d'une infection à HSV-2. Les porteurs des deux infections ont un risque plus élevé de transmettre le VIH.

WO2010/004161 A2 décrit une composition comprenant du molybdène, du GLDA comme agent chélatant, une source de radicaux péroxidants et une solution tampon. US 2007/059255A1 a trait à une composition pour traiter l'herpès; WO 2005/010774A1 vise une composition pour traiter une plaie.

L'invention a donc pour but de résoudre le problème technique consistant en la fourniture d'un procédé de préparation d'une telle composition pharmaceutique.

L'invention a également pour but de résoudre le problème technique consistant en la fourniture d'une composition pharmaceutiquement active qui soit topiquement acceptable.

L'invention a également pour but de résoudre le problème technique consistant en la fourniture d'une composition pharmaceutiquement active qui soit aisément soluble dans les solvants hydrophiles.

L'invention a encore pour but de résoudre le problème technique consistant en la fourniture d'une composition pharmaceutique active lors du passage trans-épidermique.

L'invention a encore pour but de résoudre le problème technique consistant en la fourniture d'une composition pharmaceutique qui se désactive lors du passage transcutané.

L'invention a encore pour but de résoudre le problème technique consistant en la fourniture d'une composition pharmaceutique dont la biophase est bien délimitée et atteinte.

L'invention a encore pour but de résoudre le problème technique consistant en la fourniture d'une composition pharmaceutique dont la biodisponibilité est minimale.

L'invention a encore pour but de résoudre le problème technique consistant en la fourniture d'une composition pharmaceutiquement active présentant un effet anti-réplicatif d'un virus, et en particulier de HSV.

L'invention a encore pour but de résoudre le problème technique consistant en la fourniture d'une composition pharmaceutiquement active présentant une cytotoxicité élevée pour des cellules infectée par un virus, et en particulier par HSV.

L'invention a encore pour but de résoudre le problème technique consistant en la fourniture d'une composition pharmaceutiquement active présentant peu de cytotoxicité pour des cellules saines.

L'invention a encore pour but de résoudre le problème technique consistant en la fourniture d'une composition pharmaceutiquement active qui a une efficacité prophylactique et en particulier pour HSV.

L'invention a encore pour but de résoudre le problème technique consistant en la fourniture d'une composition pharmaceutiquement active qui a une efficacité prophylactique notamment lors de la phase prodromique de l'infection et en particulier pour HSV.

L'invention a encore pour but de résoudre le problème technique consistant en la fourniture d'une composition pharmaceutiquement active qui a une efficacité prophylactique notamment par raccourcissement de la phase aiguë et en particulier pour HSV.

L'invention a encore pour but de résoudre le problème technique consistant en la fourniture d'une composition pharmaceutiquement active qui peut prévenir ou limiter le processus infectieux et en particulier celui d'HSV.

L'invention a encore pour but de résoudre le problème technique consistant en la fourniture d'une composition pharmaceutiquement active présentant une bonne stabilité lors de sa conservation à température ambiante et jusqu'à 45°C.

## Description de l'invention

Les inventeurs ont découvert qu'il était possible de résoudre les problèmes techniques mentionnés ci-dessus par la fourniture d'un mélange pharmaceutiquement ou thérapeutiquement actif comprenant au moins un peroxométallate.

On entend par « pharmaceutiquement actif » le fait que le mélange ou la composition présente une activité bénéfique dans le cadre d'un traitement thérapeutique.

Le terme « mélange » désigne une composition et ne limite pas la composition qu'il désigne à un procédé de préparation ou de fabrication particulier. Le terme « mélange » permet ici de distinguer plus facilement d'un point de vue sémantique le « mélange » de la « composition » en particulier lorsque par exemple le « mélange » est intégré dans une « composition » laquelle comprend d'autres ingrédients.

L'invention concerne avantageusement une composition ou mélange, de préférence thérapeutiquement actif par voie topique, comprenant :

- au moins un sel de métal, le métal étant choisi parmi le molybdène (Mo), le tungstène (W), le vanadium (V), l'or (Au), un lanthanide, en particulier le lanthane;
- au moins un agent chélatant choisi parmi BAPTA, EGTA et l'un quelconque de leurs mélanges;
- au moins une source de radicaux peroxydant;
- au moins un agent tampon comprenant l'acide acétique,
- ledit mélange ou ladite composition présentant un potentiel rédox de 250 à 550 millivolts, mesuré avec un pH mètre / rédox mètre combiné, équipé d'une électrode Platine / calomel étalonnée.

Le mélange selon la présente invention permet avantageusement de former un médicament, et plus particulièrement stable et actif *in situ.*

Le mélange selon la présente invention consiste en un équilibre des composés précités formant avantageusement un complexe peroxométallique y compris ses sels. Selon les inventeurs, sans vouloir être lié par la théorie, le mélange selon la présente invention permet de former une structure supramoléculaire du type réseau en équilibre stable, non permanente, qui permet de fournir au niveau extracellulaire et *in situ* un catalyseur du type réactions de Fenton et d'Haber-Weiss. Ces réactions sont dites « likes » quand elles ont lieu du fait du métal du mélange de l'invention ou d'un autre métal qui ne soit pas le fer. Une partie des substances réactives générées extemporanément par la substance active pénètre la cellule, et plus avantageusement la cellule infectée.

Selon une variante la composition comprend plusieurs sels de métal, le métal étant choisi parmi le molybdène (Mo), le tungstène (W), le vanadium (V), l'or (Au), un lanthanide, en particulier le lanthane.

Selon un mode de réalisation, le sel métallique est un sel de molybdène ou comprenant du molybdène.

Selon un mode de réalisation, le sel métallique est un sel de lanthanide ou comprenant du lanthanide.

Selon un mode de réalisation, le sel métallique est un mélange de sel de molybdène et de sel de lanthanide.

L'invention concerne avantageusement une composition ou mélange, de préférence thérapeutiquement actif par voie topique, comprenant :

- au moins un sel de métal, le métal étant choisi parmi le molybdène (Mo), le tungstène (W), le vanadium (V), l'or (Au), un lanthanide, en particulier le lanthane;
- au moins un agent chélatant choisi parmi BAPTA, EGTA et l'un quelconque de leurs mélanges;
- au moins une source de radicaux peroxydant;
- au moins un agent tampon comprenant l'acide acétique,
- ledit mélange ou ladite composition présentant un potentiel rédox de 250 à 550 millivolts, mesuré avec un pH mètre / rédox mètre combiné, équipé d'une électrode Platine / calomel étalonnée, et de préférence de 300 à 450 millivolts et encore de préférence de 300 à 420 millivolts.

De préférence, le sel métallique est présent sous forme d'oxyde(s) ou de peroxyde(s) dans le mélange. Avantageusement, les oxydes métalliques du mélange de l'invention permettent de former un intermédiaire qui est un acide métallique, et plus particulièrement un acide de Lewis.

Selon une variante, on préfère un composé métallique dont l'état d'oxydation du métal est compatible avec la réaction de Fenton-Haber-Weiss et Fenton-Haber-Weiss "like" (réaction d'oxydoréduction qui fait intervenir d'autres ions métalliques que le Fer qui, dans notre cas, est dans un contexte de chélation) métastable, à savoir, principalement, les métaux de transition et particulièrement Mo (VI), W(IV) à (VI), V(III) à (V), Au (I) à (III).

Selon une variante, on préfère un composé lanthanide pouvant former un sesqioxide de type LnOs, dont l'état d'oxydation est compatible avec la réaction de Fenton-Haber-Weiss et Fenton-Haber-Weiss "like" métastable et particulièrement La(III), Ce (III) et (IV), Nd (III) et (IV), Sm (III) et (IV), Gd(IV)..

Avantageusement, le métal du métallate ou du lanthanate est de valence maximale.

Avantageusement, le métal ou le lanthanide est oxydé à son degré d'oxydation maximal.

Selon une variante préférée, le sel métallique comprend du molybdène. Ainsi, selon une variante, le sel métallique est un sel de molybdène. Plus précisément, le mélange selon la présente invention comprend de préférence du molybdate de sodium. Selon l'invention, on préfère utiliser un sel de molybdène dans lequel la valence du molybdène est de degré VI. Ce sel permet avantageusement de procurer une stabilité importante au mélange de la composition. Par ailleurs, le molybdène, notamment sous forme de sel et par exemple de sel de sodium, par sa valence réactive (VI), son électronégativité compatible, sa très faible cytotoxicité, sa possibilité de réaction Fenton-Haber-Weiss laquelle peut être "like", est particulièrement préféré.

Selon une variante, le sel métallique formé comprend un peroxométallate, et plus particulièrement qui peut se retrouver sous la forme d'intermédiaire hydro-peroxométallate.

Avantageusement, le sel de molybdène est de préférence d'unité peroxo ou hydroperoxo. Encore avantageusement, la substance active est de type $MoO_4^{2-}$ ou sa forme hydro-peroxo (voir Oyerinde Oyeyemi F. et al., Solution structure of molybdic acid from Raman spectroscopy and DFT analysis, Inorganica Chimica Acta 361 (2008) 1000-1007. Doi:10.1016/j.ica.2007.06.025). Par exemple on peut retrouver un complexe intermédiaire de formule de type $Na_4[Mo_2O_6(chélatant)].10H_2O$ dans le mélange de l'invention.

Selon une variante, le métal ou son sel est un lanthanide ou son sel.

Selon une variante, le sel métallique formé comprend un peroxolanthanate, et plus particulièrement qui peut se retrouver sous la forme d'intermédiaire hydro-peroxolanthanate. Par exemple, on peut retrouver un complexe intermédiaire de formule de type $NaLn(chélatant).xH_2O_2.yH_2O$ (avec x et y qui est fonction du type de lanthanide (Ln) utilisé dans le mélange de l'invention.

Avantageusement, le sel de molybdène et de lanthanide, et plus particulièrement de lanthane, est de préférence d'unité peroxo ou hydroperoxo (voir Suponitskiy, Y.L., Proshina, O.P., Dyunin, A.G. et al. Thermodynamic properties of lanthanum Molybdates, Russ. J. Phys. Chem. (2016) 90:267. Doi :10.1134/S003602441602031X).

Avantageusement, l'utilisation d'un sel de molybdène permet de limiter la cytotoxicité du mélange selon l'invention, en particulier sur les hématies et lymphocytes, les épithélia, les fibroblastes, les ostéoblastes.

Un avantage d'utilisation du molybdène réside dans sa présence naturelle comme cofacteurs enzymatiques (comme par exemple la xanthine oxydase, la glycéraldéhyde-3-phosphate déshydrogénase à ferrédoxine, etc.).

Avantageusement, l'utilisation d'un sel de lanthane permet de limiter la cytotoxicité du mélange selon l'invention.

Selon une variante préférée, le métal sous sa forme de substance active joue un rôle de catalyseur de la production de substances réactives $HO_2^{\bullet}$, et éventuellement $HO'$ et $O_2^{\bullet}$.

Selon une variante, le mélange selon l'invention comprend de 0,1 à 500 $\mu$M, et de préférence de 1 à 200 $\mu$M, et encore de préférence de 5 à 150 $\mu$M de sel métallique.

Selon une variante, le mélange selon l'invention comprend de 0,1 à 100 $\mu$M, et de préférence de 1 à 50 $\mu$M, et encore de préférence de 5 à 30 $\mu$M de sel métallique de molybdène.

Selon une variante, le mélange selon l'invention comprend de 0,1 à 100 $\mu$M, et de préférence de 1 à 50 $\mu$M, et encore de préférence de 5 à 30 $\mu$M de sel métallique de lanthane.

Les termes « agents chélatants » (ou « chélatants ») correspondent à des composés chimiques aptes à former un complexe de coordination stable avec un ou plusieurs ions et plus particulièrement avec les formes ioniques du métal présent dans la composition. Le métal peut être présent sous toute forme y compris sous forme peroxydée ou hydropéroxydée. Ce complexe est dit "chélate".

L'agent chélatant de la présente invention est choisi parmi le BAPTA (1,2-bis(o-aminophenoxy)ethane-N,N,N',N'-tetraacetic acid), l'EGTA: (ethylene glycol-bis(2aminoethylether)-N,N,N',N-tetraacetic acid)), et l'un quelconque de leurs mélanges

Les agents chélatants doivent avantageusement catalyser extracellulairement, d'une part la production *in situ* de radicaux réactifs $HO_2^{\bullet}$, principalement, mais aussi $O_2^{\bullet}$ et $OH^{\bullet}$ et, d'autre part, permettre la chélation des ions calcium et métalliques dont les ions ferreux (Fe(II) ou $Fe^{2+}$) et les ions ferriques (Fe(III) ou $Fe^{3+}$) extracellulaires liés ou libres. La libération de ces réactifs est d'autant plus efficace que l'affinité des chélates pour les ions $Ca^{2+}$ et $Fe^{2+}/Fe^{3+}$ extracellulaires est importante. Ces chélates sont des composés stables présents dans le mélange pharmaceutiquement actif de l'invention et participe, outre la production préférentielle du radical $HO_2^{\bullet}$, à l'activité pharmaceutique ou thérapeutique du mélange selon la présente invention, notamment par chélation préférentielle *in situ* des ions calcium et fer. Cette chélation *in situ* permettrait aussi de réguler au niveau extracellulaire les influx calciques cellulaires, en particulier induit d'une part par une infection virale, comme par exemple par *Herpesviridae,* et d'autre part, par le stress oxydatif dû à la présence de radicaux ou de peroxocomposés.

Avantageusement, les agents chélatants ne pénètrent pas la cellule et demeurent extracellulaires.

Selon une variante préférée, les agents chélatants stabilisent des complexes peroxo et hydro-peroxomolybdates contenus dans la formulation. De tels peroxo- et hydro-peroxomolybdates peuvent être du type $(Mo_2O_6)^{4+}$ et $[Mo_4O_{12}(O_2)_2]^{4+}$.

Avantageusement, selon une variante, l'agent chélatant permet la stabilisation par chélation de l'ion substance active $MoO_4^{2-}$ lequel sous sa forme sodique a un poids moléculaire de 205.937 g par mole ou peroxomolybdate (par exemple sous la forme $MoO_3$-$BAPTAH_2^{2-}$ et/ou $MoO_3$-$EGTAH_2^{2-}$).

Selon une variante, le mélange selon l'invention comprend au moins deux agents chélatants.

Avantageusement le ou les agents chélatants lie(nt) par coordination le métallate oxydé ou peroxydé en solution.

Selon une variante, l'agent chélatant se complexe avec le métallate oxydé ou peroxydé pour former un complexe dimérique intermédiaire $[Mo_2O_6(chélatant)]^{4+}.10H_2O$ et/ou de la forme peroxotetramolybdate(VI) $[Mo_4O_{12}(O_2)_2]^{4+}$-Chélatant, lorsque le sel métallique est un sel de molybdène.

Selon une variante, l'agent chélatant se complexe avec le métallate oxydé ou peroxydé pour former un complexe $[W_2O_6(chélatant)]^{4+}.8H_2O$, lorsque le sel métallique est un sel de tungstène.

Selon une variante, l'agent chélatant se complexe avec un lanthanate oxydé ou peroxydé pour former notamment un complexe $[La(O_2)$-Chélatant$]^{2+}$. $6H_2O$ lorsque le sel de lanthanide est un sel de lanthane.

Avantageusement, l'agent chélatant est compatible avec la capture stérique des complexes oxydés et/ou peroxydés du métallate. Plus particulièrement, la taille de sa "poche" N---N qui sépare les résidus carboxyliques impliqués dans la coordination ainsi que le repliement stérique de la chaine carbonée de cette poche qui doit être avantageusement compatible avec la capture des complexes métalliques intermédiaires, notamment sous forme oxydée et/ou peroxydée. Avantageusement, l'agent chélatant est compatible avec les charges électroniques du métallate et avec sa structure orbitale atomique externe. BAPTA et/ou l'EGTA autorise une meilleure stabilité des précurseurs ainsi qu'un meilleur rendement de synthèse.

Les pKa des 4 groupements carboxyliques de BAPTA sont: pK1 et pK2 <4 (BAPTA insoluble), pK3 = 5.47, et pK4 = 6.36 ; et de EGTA sont : pK1 = 2.00, pK2 = 2.65, pK3 = 8.85, et pK4 = 9.46.

Avantageusement, les pKa du/des agent(s) chélatant(s) sont compatibles avec le pH de la solution inventive.

Avantageusement, les pKa du/des agent(s) chélatant(s) sont compatibles avec le pKa de l'acide métallique ou peracide métallique formé intermédiairement dans la solution inventive.

Avantageusement, les pKa du/des agent(s) chélatant(s) sont compatibles avec le pH de la région infectée, comme par exemple les épithélia.

Avantageusement, les agents chélatants, BAPTA et EGTA, sont faiblement cytotoxiques.

Le sel métallique et les agents chélatants sont présents en quantité et ratio suffisant pour la production de complexes {peroxo-métallate - agent(s) chélatant(s)}.

De préférence, au moins un des agents chélatants présents a une affinité supérieure pour le calcium que pour le métal utilisé dans le mélange de l'invention. Ceci permet avantageusement une substitution des complexes chélates du mélange de l'invention par un complexe agent chélatant-calcium *in vivo*. Avantageusement, la concentration du ou des chélates est compatible avec la concentration du $Ca^{2+}$ extracellulaire (2-3mM dans un plasma humain), c'est-à-dire que le ou les agents chélatants présents dans le mélange de l'invention sont en quantités suffisantes pour complexer efficacement d'un point de vue thérapeutique la quantité de calcium extracellulaire présente.

Avantageusement, le ou les chélates présents ont une affinité supérieure pour le fer ($Fe^{2+}/Fe^{3+}$) que pour le métal utilisé dans le mélange de l'invention. Ceci permet avantageusement une substitution des complexes chélates du mélange de l'invention par un complexe agent chélatant-$Fe^{2+}/Fe^{3+}$, *in vivo*.

Selon une variante, le mélange selon l'invention comprend de 0,0001 à 1 mM d'agents chélatants.

Selon une variante, le mélange selon l'invention comprend de 0,1 à 100 $\mu$M, et de préférence de 1 à 50 $\mu$M, et encore de préférence de 5 à 20 $\mu$M de BAPTA. Selon une variante, le mélange selon l'invention comprend de 1 à 80 $\mu$M, et encore de préférence de 20 à 80 $\mu$M de BAPTA.

Selon une autre variante, le mélange de l'invention comprend de 0,1 à 1 mM, et de préférence de 50 à 800 $\mu$M, et encore de préférence de 200 à 700 $\mu$M d'EGTA. Selon une variante, le mélange de l'invention comprend de 200 à 2000 $\mu$M, et encore de préférence de 600 à 1400 $\mu$M d'EGTA.

Selon une autre variante, le mélange l'invention comprend du BAPTA et EGTA présent à une concentration de 0,0001 à 1 mM, et de préférence de 5 à 20 $\mu$M et 200 à 700 $\mu$M, respectivement.

Selon une variante, le sel de molybdène et les agents chélatants sont présents selon un ratio 10/1 à 1/100 (ratio : sel de Mo/agent chélatant, exprimé en concentrations molaires).

Selon une variante, le sel de lanthane et les agents chélatants sont présents selon un ratio 10/1 à 1/100 (ratio : sel de La/agent chélatant, exprimé en concentrations molaires).

Selon une variante, le sel de molybdène et BAPTA sont présents selon un ratio 3/1 à 1/10, exprimé en concentrations molaires.

Selon une variante, le sel de molybdène et EGTA sont présents selon un ratio 1/10 à 1/70, exprimé en concentrations molaires.

Selon une variante, le sel de lanthane et BAPTA sont présents selon un ratio 3/1 à 1/10, exprimé en concentrations molaires.

Selon une variante, le sel de lanthane et EGTA sont présents selon un ratio 1/10 à 1/70, exprimé en concentrations molaires.

Selon une variante, la source de radicaux est principalement $H_2O_2$.

Selon une variante, une ou plusieurs sources secondaires de radicaux peuvent être ajoutées ou être synthétisées minoritairement *in situ*.

Selon une variante, $H_2O_2$ est une solution aqueuse comprenant $H_2O_2$ à 110 volumes ou 30% ou 8.82M.

Avantageusement, la source d'$H_2O_2$ ne comprend pas d'agent stabilisant choisi parmi : les acides pyridine-carboxyliques, comme par exemple l'acide picolinique ; les acides phosphoniques et aminophosphoniques ; les amides ou amines substitués ; les acides alkylsulfoniques ; et leurs mélanges.

Selon une variante, la source principale de radicaux peroxydant est le peroxyde d'hydrogène. Ainsi, selon cette variante, le peroxyde d'hydrogène est présent à une concentration allant de 200 à 600 mM, de préférence de 300 à 500 mM, et encore de préférence de 340 à 450 mM.

Selon une variante, le mélange selon l'invention comprend une proportion de peroxyde d'hydrogène allant de 30 mM à 4,4 M, et de préférence de 150 mM à 3 M, et de préférence de 235 mM à 1,8 M, et encore de préférence de 260 mM à 440 mM. Selon une variante, le mélange de l'invention ne comprend pas d'ajout extemporané de peracide.

Avantageusement, la quantité (ou la concentration) de peroxyde d'hydrogène ($H_2O_2$) est dosée par titrimétrie. Typiquement, on peut utiliser un dosage à l'aide d'une solution de permanganate de potassium.

La concentration en peroxyde d'hydrogène peut également être dosée par spectroscopie UV.

Avantageusement, on utilise un agent tampon comprenant l'acide acétique afin de contrôler précisément le pH du mélange selon l'invention.

Avantageusement, on utilise un agent tampon comprenant l'acide acétique afin de contrôler précisément le pH lors de l'application *in situ* de la solution selon l'invention.

De préférence, l'agent tampon ne comprend pas les acides suivants : acides carboxyliques hydroxylés (par ex. l'acide malique) ou polycarboxyliques (par ex. les acides citrique et isocitrique, l'acide malonique); les acides carboxyliques de poids moléculaires supérieur à 100 g/mol. et en particulier les acides carboxyliques à longues chaine (supérieure à C6) du fait des déstabilisations membranaires cellulaires qu'ils peuvent induire, ainsi que les acides carboxyliques aliphatiques insaturés (acide sorbique, par exemple) du fait de leur possibilité de peroxydation de type alcènoïque cytotoxique.

Selon une variante, la présente invention inclut la présence de certains acides générés *in situ* lors de la synthèse, de préférence en faible quantité (comme par exemple l'acide peracétique). Ils servent de source accessoire de radicaux au moment de la fabrication de l'invention et de l'utilisation thérapeutique de l'invention.

Selon une variante, on préfère un agent tampon présentant un pKa au niveau du pH physiologique (peau et muqueuses), c'est-à-dire un pH par exemple compris entre 4,4 5,0, et compatible avec le pKa de l'acide métallique oxydé et/ou peroxydé dont la forme est ionisée (par ex. $MoO_4H_2$, pk1 = 3.61 et $pKa_2$ = 3.89).

De préférence, l'agent tampon est présent dans le mélange de l'invention à une concentration comprise entre 1 et 500 mM, et de préférence entre 10 et 200 mM, encore de préférence entre 50 et 100 mM.

Le ratio des concentrations molaires en peroxyde d'hydrogène et en agent tampon est compris entre 2 et 9 et préférentiellement entre 3 et 8.

Avantageusement, le mélange est tamponné pour obtenir un pH de 4.4 à 5.0 lequel est compatible i) pour un contact de l'invention avec la peau et les muqueuses, ii) la stabilité de la substance active $MoO_4^{2-}$ en présence d'$H_2O_2$, iii) la réaction catalytique de Fenton-Haber-Weiss "like" ou non et, iv) la production *in situ* d'espèces radicalaires principalement de type HOO'.

Selon une variante, le mélange de la présente invention comprend comme agent tampon autorisant un pH 4,0 à 5,2, préférentiellement de 4,4 à 5,0.

Avantageusement, le mélange présente un potentiel rédox de 350 à 450 mV, et de préférence de 350 à 420 mV.

L'efficacité rédox du mélange de l'invention peut être aussi estimée par l'oxydation *in vitro* de la quercétine par les ions fer(III) ou $Fe^{3+}$. On peut notamment utiliser pour cela une méthode de dosage dite « méthode quercétine », (adaptée par exemple des études de El Hajji H. et al (2006) "Interactions of quercetin with iron and copper ions: Complexation and autoxidation", Free Radic. Res., 40(3), 303-320 et Balcerzak M. et al (2008) "Sélective determination of Fe(III) in Fe(II) samples by UV-spectrophotometry with the aid of quercetin and morin", Acta Pharm., 58, 327-334*).*

La présente invention concerne également un procédé de préparation d'un mélange tel que défini selon la présente invention telle que décrite dans les revendications. Plus spécifiquement, la présente invention concerne un procédé de préparation d'un mélange selon l'invention comprend (i) la préparation d'une solution tampon (ST) comprenant un agent tampon présentant un pH acide, (ii) la préparation d'une solution d'un complexe métallique (SC) comprenant un sel d'oxyde métallique, (iii) la préparation d'une première solution initiale (Si1) comprenant du peroxyde d'hydrogène, (iv) la préparation d'une seconde solution initiale (Si2) par mélange de la solution ST avec la solution Si1, (v) la préparation d'une solution (S1) comprenant un composé peroxo-métallique par mélange de la solution SC avec la solution Si2, (vi) la préparation d'une solution S2 par ajustement du pH de la solution S1 avec une base, le pH de la solution S2 étant plus basique que le pH de la solution ST, (vii) l'ajout à la solution S2 d'un ou plusieurs agents chélatants, (viii) l'ajustement éventuel du pH, (ix) l'ajustement éventuel du volume de la solution finale, l'obtention d'un mélange pharmaceutiquement actif tel que défini selon l'invention .

Selon une variante, lorsque deux sels d'oxydes métalliques ou plus sont utilisés, avec des oxydes métalliques différents, la préparation d'une solution d'un complexe métallique (SC) peut consister en la préparation de solutions distinctes pour chaque sel d'oxyde métallique puis le mélange de ces solutions simultanément ou successivement avec la solution Si2 pour préparer une solution S1.

De préférence, la préparation du mélange selon la présente invention est réalisée selon un procédé de synthèse catalytique. La synthèse catalytique du mélange selon l'invention est typiquement séquentielle. Avantageusement, le procédé de l'invention comprend la séquence des étapes (iii) => (iv) => (v) => (vi) => (vii) => (viii) => (ix).

Avantageusement, le procédé de préparation comprend un suivi par analyse spectrale (par exemple par analyse des spectres IR, UV et/ou visible) à une ou plusieurs des étapes de préparation, et de préférence lors de toutes les étapes de préparation.

Selon une variante, la première solution initiale Si1 présente un premier potentiel rédox supérieur au potentiel rédox du mélange selon l'invention.

De préférence, suite à l'étape (ix), il n'y a pas de dilution du volume du mélange de manière à éviter les déplacements des équilibres chimiques du mélange selon la présente invention.

Il est évidemment avantageux d'utiliser des substances ou composés de pureté suffisamment satisfaisante à titre de matières premières. Il faut notamment que les différentes substances ou composés présentent une compatibilité avec une utilisation thérapeutique.

Selon une variante, on peut réaliser une spectrométrie IR-FT, RMN-H et/ou de masse sur le mélange selon l'invention produit pour s'assurer de la conformité de la composition du mélange aux exigences industrielles, et en particulier pour une utilisation thérapeutique pharmaceutique. On réalise de préférence également une spectrométrie IR-FT sur les composants précurseurs du mélange de l'invention.

Selon une variante, on peut contrôler le pH à une ou plusieurs, voire l'ensemble des étapes de préparation.

Selon une variante, on peut contrôler le potentiel rédox à une ou plusieurs, voire l'ensemble des étapes de préparation.

Selon une variante, on peut contrôler le mélange de l'invention par densimétrie, et/ou spectrophotométrie UV, à une ou plusieurs, voire l'ensemble des étapes de préparation.

Selon une variante, on peut doser l'efficacité rédox du mélange de l'invention à l'aide de la méthode quercétine (dosage en spectrophotométrie UV de l'oxydation *in vitro* de la quercétine par le $Fe^{3+}$ présent ou produit *in vitro*).

Avantageusement, le peroxyde d'hydrogène est présent dans la solution Si1 à une concentration allant de 200 à 600 mM, de préférence de 300 à 500 mM, et encore de préférence de 330 à 460 mM.

Selon un mode de réalisation, le matériel utilisé est métallique et est de préférence en inox, éventuellement passivé.

Selon un mode de réalisation, on peut re-passiver le matériel préalablement à la préparation du mélange de l'invention par exemple en mettant le matériel (qui sera en contact avec le mélange ou ses précurseurs) en contact avec une solution de peroxyde d'hydrogène.

Il est préférable de réaliser un dosage de la source de peroxyde d'hydrogène afin de s'assurer de la quantité de peroxyde d'hydrogène présent et à mettre en jeu dans la préparation du mélange de l'invention. Ce dosage peut se réaliser par exemple par titrimétrie ou par spectrophotométrie UV (en général à 240 nm).

Avantageusement, le pH de la solution tampon ST est de 4,4 à 5,0, et de préférence de 4,7 à 4,8.

Avantageusement, l'agent tampon de la solution ST est un tampon acide carboxylique/carboxylate, et la solution ST présente un ratio peroxyde d'hydrogène/carboxylate compris entre 20/1 et 1/1, et de préférence d'environ 5/1.

Avantageusement, le ratio de l'acide acétique (AcOH) avec son sel ajouté l'acétate en quantité limitante, autorise la production la plus faible d'acide peracétique (AcOOH). Avantageusement, dans un procédé de préparation selon l'invention, la production d'acide peracétique est minimale et l'influence catalytique de celui-ci est très faible.

La préparation de la première solution initiale Si1 comprend la préparation d'une solution aqueuse d'acide carboxylique, et de préférence d'acide acétique puis l'ajout de peroxyde d'hydrogène.

De préférence, la première solution initiale Si1 présente un pH de 2,5 à 4.6, et de préférence de 2,7 à 4.2. Avantageusement, la première solution initiale Si1 présente un potentiel rédox de 400 à 550 mV, et de préférence de 420 à 500 mV.

Avantageusement, le procédé comprend une mesure du potentiel rédox aux étapes (iii) à (ix), et de préférence comprend également une mesure du pH aux étapes (iii) à (ix).

Selon une variante, la seconde solution initiale Si2 présente un pH de 2,5 à 3,2, et de préférence d'environ 2,9. Selon une variante, la solution initiale Si2 présente un potentiel rédox supérieur au potentiel rédox de la première solution initiale Si1. Le potentiel rédox de la seconde solution initiale Si2 peut-être de 420 à 570 mV, et de préférence de 440 à 520 mV.

Selon une variante, on introduit la solution tampon ST dans la première solution initiale Si1.

Selon un mode de réalisation, la concentration en peroxyde d'hydrogène dans la seconde solution initiale Si2 va de 300 à 500 mM, et de préférence de 260 à 440 mM.

Avantageusement, dans l'étape (iv) le pH est maintenu en deçà de la limite admissible de pH pour la réaction catalytique de synthèse du mélange selon l'invention. On utilise avantageusement un agent tampon à l'étape (iv)

Avantageusement, dans l'étape (iv) la production *in situ* de l'acide peracétique générée est minimale.

Avantageusement, dans l'étape (iv) le potentiel rédox de la solution Si1 de l'étape (iii) est augmenté, par exemple en passant d'une valeur de 440 à 460mV à une valeur de 460 à 480mV de l'étape (iv).

En suivant la concentration de peroxyde d'hydrogène présent lors de la préparation du mélange de l'invention, on observe une production peroxydique lors de son introduction, à savoir la mise en contact du peroxyde d'hydrogène avec la solution tampon (étape (iv)). Avantageusement, l'ajout de peroxyde d'hydrogène est réalisé dans une solution aqueuse préalablement tamponnée à un pH acide. Ainsi, pour la préparation de la première solution initiale Si1 le peroxyde d'hydrogène est ajouté dans une solution à pH acide, et par exemple à pH de 2,7 à 4,2.

Avantageusement, l'étape (iv) conduit à déplacer l'équilibre vers une production *in situ* d'$H_2O_2$ avec destruction de l'acide peracétique éventuellement généré par les équilibres chimiques.

Selon une variante, on introduit la solution tampon SC dans la solution S1.

Selon une variante, la solution S1 comprenant un complexe peroxométallique présente un pH de 2,5 à 3,5, et de préférence de 2,8 à 3,0.

Selon un mode de réalisation, le potentiel rédox de la solution S1 comprenant un complexe peroxométallique va de 400 à 550 mV, et de préférence de 440 à 500mV.

Selon un mode de réalisation, la concentration de peroxyde d'hydrogène dans la solution S1 comprenant un complexe peroxométallique va de 250 à 500 mM, et de préférence de 320 à 450 mM.

A l'étape (v), le sel de molybdate ajouté est sous la forme de l'acide molybdique $MoO_3.H_2O$ et $MoO_3.2H_2O$.

En conditions non inventive, la synthèse à l'équilibre de la substance active $MoO_4^2$, à température ambiante, sans catalyseur et sans ajout d'acide fort ($H_2SO_4$, par exemple) demanderait plusieurs jours.

Selon un mode de réalisation, le pH de la solution S2 est tamponné à une valeur de préférence comprise entre 4,4 et 5,0, et par exemple de 4,5 à 5.0. Avantageusement, à ce pH tamponné, c'est la forme instable $MoO_4^{2-}$ qui prédomine (état d'oxydation Mo(VI)) et le peroxyde d'hydrogène est stabilisé. Le peroxyde d'hydrogène est souvent stabilisé industriellement par un tampon phosphate. Il peut se former un composé intermédiaire instable de phosphomolybdate (jaune) qui disparaît à l'équilibre, rendant $H_2O_2$ plus réactif.

Selon une variante, le potentiel rédox de la solution S2 va de 300 à 450 mV, et de préférence de 360 à 410mV.

Les oxydes de molybdène Mo(VI) en solution aqueuse et au pH de synthèse du mélange de l'invention peuvent exister sous différentes structures moléculaires : $MoO_4^{2-}$ et $[MoO_6^{2-}]$, $[Mo_2O_3(O_2)_4(H_2O)_2]^{2-}$, mais aussi sous des formes polynucléaires réactives (comme des structures complexes binucléaires oxo-Mo(VI) (Dement'ev I.A. et al (2007)" Mononuclear, polynuclear, and cluster complexes of molybdenum and their reactions as models of biochemical systems and processes", Russ. J. Gen. Chem., 77(5), 822-843)).

Avantageusement, les conditions de préparation permettent la formation *in situ* de peroxo- et d'hydro-peroxo-molybdate lorsque le sel métallique utilisé est un sel de molybdène. Ces derniers sont de type : $[Mo_2O_3(O_2)_4(H_2O)_2]^{2-}$. Selon une variante, on préfère un excès molaire de peroxyde d'hydrogène supérieur à 1000 fois, et de sel métallique, et en particulier de préférence supérieure à 10000 fois, et encore de préférence à 15000, par rapport au Mo(VI).

Avantageusement, le ou les agents chélatants ainsi que le pH de synthèse limitent les déplacements de la réaction du type « réducteur de Jones » consistant à la réduction de l'ion métallate, avantageusement à savoir le molybdate, à un état d'oxydation bas correspondant au métal molybdène. Selon une variante, ce déplacement est limité dans un milieu aqueux faiblement acide, typiquement au pH compris entre 4,4 et 5,0, et par exemple de 4,5 à 4,7.

De préférence, lorsque plusieurs agents chélatants sont présents, on prépare des solutions indépendantes d'agents chélatants, chaque solution comprenant un agent chélatant spécifique, choisi parmi BAPTA et/ou EGTA.

Selon une variante préférée, l'ajout des agents chélatants se réalise par ordre de pKa croissants.

On peut par exemple préparer d'une part une solution comprenant un agent chélatant du type BAPTA et d'autre part une solution d'agent chélatant du type EGTA. On ajoute de préférence BAPTA puis EGTA.

Avantageusement, le ou les agents chélatants permettent *in situ,* c'est-à-dire après application topique notamment, la réaction de Fenton-Haber-Weiss avec notamment une constante de dissociation $Kd_{chélate}$ : $Fe^{2+}/Fe^{3+} > Ca^{2+}$.

Avantageusement, le ou les agents chélatants permettent extracellulairement *in situ,* l'auto-entretien de la production radicalaire réactive HOO' principalement, et minoritairement $O_2^•$ et $HO^•$.

Selon une variante, on peut d'abord ajouter le BAPTA à la solution S2 pour obtenir une solution S3 résultante. La solution S3 présente par exemple un pH allant de 4,0 à 5,0, et de préférence de 4,3 à 4,8. La solution S3 présente par exemple un potentiel rédox essentiellement identique à celui de la solution S2. En d'autres termes, le potentiel rédox ne varie pas lors de l'ajout de l'agent chélatant du type BAPTA. Selon une variante, le potentiel rédox de la solution S3 va de 250 à 450 mV, et de préférence de 300 à 400mV. Avantageusement, on réalise une dissolution préalable de BAPTA dans un faible volume de solution S2 avant mise en contact avec le reste de la solution S2.

De préférence, la concentration de BAPTA est dans un ratio molaire de 1/1 à 1/3 et de préférence de 1/2, par rapport au sel de molybdène.

Par exemple, la solution S3 présente une concentration de peroxyde d'hydrogène de 250 à 500 mM, et de préférence de 320 à 440 mM.

On peut avantageusement ajouter au moins un autre agent chélatant du type EGTA dans la solution S3 pour obtenir une solution S4 résultante. Selon une variante, la solution S4 présente un pH allant de 4,0 à 5,0, et de préférence de 4,3 à 4,7. Selon une variante, le potentiel rédox de la solution va de 300 à 400 mV, et de préférence de 350 à 390 mV.

De préférence, la concentration de l'EGTA est dans un ratio molaire de 10/1 à 50/1 et de préférence d'environ 25/1, par rapport au sel de molybdène.

Par exemple, la solution S4 présente une concentration de peroxyde d'hydrogène de 250 à 550 mM, et de préférence de 320 à 470 mM.

Selon une variante, le pH à l'étape (viii) est ajusté par une solution basique, par exemple du type hydroxyde de sodium, de préférence concentrée, par exemple à une concentration de 8 à 15 M. Avantageusement, le pH à l'étape (viii) peut être ajusté à une valeur de 4,4 à 5.0, et de préférence d'environ 4,6.

Selon une variante, le pH du mélange de l'invention va de 4,50 à 4,70. Avantageusement, le potentiel rédox du mélange de l'invention va de 320 à 390 mV.

Selon une variante préférée, le procédé comprend une diminution du potentiel rédox à l'étape (vi). Une diminution du potentiel rédox peut par exemple être de 50 à 150mV, et typiquement être d'environ 90mV.

De préférence, le potentiel rédox est sensiblement constant suite à la diminution du potentiel rédox de l'étape (vi).

Avantageusement, le mélange selon la présente invention est stable dans le temps à la conservation, par exemple à température ambiante ou jusqu'à une température de 45 °C pendant six mois. Il est recommandé de conserver le mélange à l'obscurité et à l'abri de l'humidité.

Le pH est mesuré à l'aide d'un pH mètre étalonné et à compensation de température, avec une électrode combinée KCl 4M / AgCl

Le potentiel rédox est mesuré avec un pH mètre / rédox mètre combiné, équipé d'une électrode Platine / calomel étalonnée. Typiquement, cette mesure est réalisée à température ambiante, c'est à dire à 20°C et à pression atmosphérique, c'est-à-dire 101325 Pa.

On évalue de préférence la concentration de peroxyde d'hydrogène dans les solutions précitées par spectroscopie UV selon l'équation dérivée de la loi de Beer-Lambert : $A_{240nm} = 43{,}6 \times l \times [H_2O_2]$ avec l en centimètre et $[H_2O_2]$ en mole (Noble R.W. et al (1970) "The reaction of ferrous horseradish peroxidase with hydrogen peroxide", J. Biol. Chem., 245(9), 2409-2413).

Selon une variante spécifique, le composé peroxo-métallique de la solution S1 est un composé peroxo-molybdique.

Le mélange de l'invention comprend donc selon cette variante un composé peroxométallique. Selon une variante préférée, le mélange de l'invention comprend un complexe peroxomolybdique.

Selon une variante, la solution comprend du molybdate de sodium sous la forme Mo(VI). Avantageusement, au pH des solutions d'invention, le sel est sous la forme catalytique $MoO_3\text{-}BAPTA^{3-}$ principalement et $MoO_3\text{-}EGTA^{3-}$ principalement, les deux sources de la substance active $MoO_4^{2-}$.

Selon un mode de réalisation particulier, le mélange selon l'invention comprend un sel, de préférence de sodium, de molybdène, un mélange de deux agents chélatants, de préférence du type BAPTA et EGTA, du peroxyde d'hydrogène et un agent tampon acétate (acide acétique/acétate de sodium, par exemple).

Selon une variante spécifique, le mélange selon la présente invention comprend :

- sel de Molybdate, et par exemple Molybdate de sodium;
- BAPTA;
- EGTA;
- $H_2O_2$,
- CH3COOH/CH3COONa;
- $E_{rédox}$ de 250 à 550 mV, et de préférence de 300 à 450 mV, et encore de préférence de 300 à 420mV;
- pH 4.4 - 5.0.

Selon une variante spécifique, le mélange selon la présente invention comprend :

- sel de Molybdate, et par exemple Molybdate de sodium;
- sel de Lanthane, et par exemple Nitrate de Lanthane;
- BAPTA;
- EGTA;
- $H_2O_2$,
- CH3COOH/CH3COONa;
- $E_{rédox}$ de 250 à 550 mV, et de préférence de 300 à 450 mV, et encore de préférence de 300 à 420mV;
- pH 4.4 - 5.0.

Avantageusement, les composés du mélange sont en quantités et conditions de pH et potentiel rédox nécessaires pour former un complexe peroxomolybdate ou hydro-peroxomolybdate où le molybdène est de valence VI.

Selon un mode de réalisation particulier, le mélange de l'invention comprend au moins un agent chélatant et au mieux et au minimum deux, et de préférence de 5 à 20 μM et 200 à 700 μM, respectivement.

Selon un mode de réalisation particulier, le mélange de l'invention comprend de 340 mM à 450 mM de donneur principal de radicaux comme $H_2O_2$.

L'invention concerne en particulier une composition, définie selon les revendications, pour son utilisation dans une méthode de traitement thérapeutique.

L'invention concerne aussi une méthode de traitement thérapeutique comprenant l'administration, avantageusement par application topique, à un sujet en ayant besoin d'une quantité thérapeutiquement efficace d'une composition pharmaceutique selon l'invention.

L'invention concerne également un procédé de préparation d'une composition pharmaceutique selon l'invention pour son utilisation dans une méthode de traitement thérapeutique.

L'invention concerne en particulier des compositions pharmaceutiques comprenant ou consistant du mélange selon l'invention.

L'invention concerne ainsi une composition pharmaceutique à usage topique, caractérisée en ce qu'elle comprend un mélange thérapeutiquement actif, tel que défini selon l'invention, ou susceptible d'être obtenu selon un procédé tel que défini selon l'invention.

Avantageusement, la composition pharmaceutique selon l'invention comprend de 0,001 à 5 mM, de préférence de 0,01 à 2 mM, et encore de préférence de 0,02 à 1 mM du mélange pharmaceutiquement actif.

L'invention concerne en particulier une composition pharmaceutique à usage topique selon l'invention pour son utilisation dans une méthode de traitement thérapeutique d'une infection virale, et en particulier impliquant un virus de la famille *Herpesviridae.*

L'invention concerne en particulier une composition pharmaceutique à usage topique selon l'invention pour son utilisation dans une méthode de traitement thérapeutique d'une infection impliquant HSV-1 et/ou HSV-2.

L'invention concerne en particulier une composition pharmaceutique à usage topique selon l'invention pour son utilisation dans une méthode de traitement thérapeutique anti-inflammatoire.

L'invention concerne en particulier une composition pharmaceutique à usage topique selon l'invention pour son utilisation dans une méthode de traitement thérapeutique préventif ou curatif.

L'invention concerne aussi une composition pharmaceutique, définie selon les revendications, comprenant du molybdène, au moins un agent chélatant et une source de radicaux peroxydant pour son utilisation dans une méthode de traitement thérapeutique d'un herpès. Cette composition est définie selon l'un quelconque des modes de réalisation, variantes, avantages, préférences, ou exemples de l'invention, pris seul ou selon l'une quelconque de leurs combinaisons.

Le terme « selon l'invention » fait référence à l'un quelconque des modes de réalisation, variantes, avantages, préférences, ou exemples de l'invention, pris seul ou selon l'une quelconque de leurs combinaisons.

Typiquement, les compositions pharmaceutiques à usage topique selon l'invention contiennent des excipients et en particulier des excipients agréés par la pharmacopée.

Par traitement thérapeutique anti-infectieux, on entend à la fois un traitement thérapeutique préventif, un traitement prophylactique et un traitement curatif permettant de limiter par exemple l'infection en cas de contact contaminant, l'occurrence d'une maladie, sa phase aigüe avec, partiellement ou non éradication de l'agent causal et/ou limitation de sa dissémination.

Avantageusement, le mélange actif ou la composition pharmaceutique selon la présente invention est active *in situ* lors de son application.

Avantageusement, le mélange actif ou la composition pharmaceutique selon la présente invention est actif lors du passage trans-épithélial et de préférence ne permet pas ou de façon limitée son passage transcutané.

Avantageusement, le mélange actif ou la composition pharmaceutique selon la présente invention est désactivé lors du passage transcutané.

Selon une variante, le mélange selon l'invention ou une composition en comprenant est appliqué par voie topique au niveau orofacial.

Selon une variante, le mélange selon l'invention ou une composition en comprenant est appliqué par voie topique au niveau génital ou anal.

Selon une variante, le mélange selon l'invention ou une composition en comprenant est appliqué par voie topique sur la peau.

Selon une variante, le mélange selon l'invention ou une composition en comprenant est appliqué par voie topique sur une muqueuse.

Selon une variante, des excipients agréés par les pharmacopées européenne (Ph. *Eur.),* Nationales (*USP, JP, IP, Ph. Helv., Ph.Belg., Ph Fr., BP, DAB, OAB, notamment*) et internationale *(Ph. Int.;* OMS), peuvent être introduits lors de la fabrication de l'invention et/ou dans le « mélange »noté « Mélange 1 ».

Les mélanges et compostions de l'invention sont particulièrement adaptés pour une activité pharmaceutique chez l'être humain.

Avantageusement, le mélange actif de l'invention permet de mettre en oeuvre des réactions de type Fenton-Haber-Weiss et Fenton-Haber-Weiss "like", et de générer préférentiellement le radical $HO_2^\bullet$ et minoritairement les radicaux $O_2^\bullet$, $HO^\bullet$.

Selon les inventeurs, la perméation de la substance réactive $HOO^\bullet$, est suffisante pour induire de façon limitée et contrôlée l'apoptose cellulaire.

Selon les inventeurs, la perméation de la substance réactive $HOO^\bullet$, est suffisante pour avoir de façon efficace et contrôlée un effet antiréplicatif viral.

Selon l'invention, de préférence on utilise le potentiel rédox du mélange de l'invention pour limiter dans un cadre étroit les concentrations des précurseurs des substances actives radicalaires de façon à générer un juste équilibre entre : un effet anti-réplicatif / une cytotoxicité modérée pour la cellule saine / une cytotoxicité importante pour la cellule infectée / un effet préventif à l'infection.

Avantageusement, le mélange thérapeutiquement ou pharmaceutiquement actif selon l'invention permet de générer principalement et préférentiellement l'espèce radicalaire réactive $HOO^\bullet$

Ainsi, selon la présente invention le calcium extracellulaire *in vivo* est le principal facteur déclencheur de la production d'$HOO^\bullet$. Il s'agit par exemple principalement d'une action au niveau du complexe $2Mo_{(VI)}\text{-}BAPTA_2(H_2O_2)$ lorsque le sel de molybdène et le BAPTA sont mis en jeu dans le mélange de l'invention. Il s'agit par exemple principalement d'une action au niveau du complexe $2Mo_{(VI)}\text{-EGTA}_2(H_2O_2)$ lorsque le sel de molybdène et l'EGTA sont mis en jeu dans le mélange de l'invention.

Avantageusement, la production dans le milieu extracellulaire d'HOO$^\bullet$ autorise des modifications extracellulaires (oxydations de résidus aminoacides extra membranaires, par exemple) pouvant ainsi limiter l'infection virale par modification de la reconnaissance cellulaire.

Avantageusement, la production dans le milieu extracellulaire d'HOO$^\bullet$ ainsi que sa perméation cellulaire sont compatibles avec sa une durée de vie longue ($\geq$ 1 sec.) et sa grande pénétration cellulaire *vs.* OH$^\bullet$ ou $O_2^\bullet$ (1msec. et pénétration de l'ordre du nm). Avantageusement, cette substance HOO' intervient dans la réaction d'Haber Weiss et de la réaction de Fenton ("like" ou non) au niveau extracellulaire et intracellulaire et permet un auto-entretien de celles-ci.

Avantageusement, le mélange contenant Mo(IV) selon la présente invention présente un pH de 4,4 à 5.0. Cela permet notamment d'optimiser la constante de vitesse $k_{obs}$ (M$^{-1}$.s$^{-1}$) de la décomposition $HO_2^\bullet/O_2^\bullet$.

Avantageusement, la modification du potentiel rédox intracellulaire par la mise en contact avec la composition selon l'invention permet un équilibre apoptotique/anti-apoptotique. Plus particulièrement, cet équilibre apoptotique/anti-apoptotique est fonction notamment de l'existence d'une infection par HSV et principalement par HSV-1 et -2.

Selon une variante, le mélange de l'invention est actif sélectivement sur les cellules infectées par HSV et principalement par HSV-1 et -2.

Ainsi, Il existe un étroit équilibre entre les voies du stress induites par l'infection à HSV, dont l'influx calcique, et la régulation de l'apoptose.

Cet équilibre est perturbé par la présence de radicaux intra-cytoplasmiques et des modifications du potentiel rédox cellulaire. Ceci induit les mêmes voies de stress cellulaire qu'une infection par HSV, les mêmes influx calciques et les mêmes inductions de voies métaboliques, ces mêmes voies étant rétro-régulées vers un autre équilibre anti-apoptotique. Ceci supporte l'effet thérapeutique du mélange selon l'invention ou des compositions le comprenant.

Selon une variante, la composition pharmaceutique ou le mélange de l'invention est utilisé dans une méthode de traitement thérapeutique d'un herpès labial et orofacial.

Selon une variante, la composition pharmaceutique ou le mélange selon l'invention est utilisé dans une méthode de traitement thérapeutique d'un herpès génital et anal.

Selon un mode de réalisation, le mélange actif selon l'invention a une efficacité anti-infectieuse (action directe modérée à faible sur le virus, récepteur viraux à la cellule comme candidats possibles), anti-reconnaissance et internalisation (action importante, récepteurs cellulaires au virus). Ces efficacités sont externes à la cellule et limitent l'infectivité virale. Ainsi, le mélange actif selon l'invention présente une action prophylactique. Ainsi encore, le mélange actif selon l'invention présente une efficacité anti-infectieuse.

Selon une variante, la composition pharmaceutique ou le mélange selon l'invention est utilisé dans un traitement thérapeutique d'une population exprimant HSV sous sa forme enveloppée, réplicative ou infectante.

Selon une variante, la composition pharmaceutique selon l'invention ou le mélange est utilisé dans un traitement thérapeutique d'une population cellulaire ou d'un tissu exprimant HSV-1 (Kessler H.H. et al (2000) "Détection of Herpes Simplex Virus DNA by real-time PCR", J Clin Microbiol., 38(7), 2638-2642).

Selon une variante, la composition pharmaceutique selon l'invention ou le mélange est utilisé dans un traitement thérapeutique d'une population cellulaire ou d'un tissu exprimant HSV-2 (Kessler H.H. et al (2000) "Détection of Herpes Simplex Virus DNA by real-time PCR", J Clin Microbiol., 38(7), 2638-2642).

Avantageusement, le mélange actif selon l'invention a une efficacité anti-réplicative induite du fait d'un contact préalable à l'infection avec une cellule ou un tissu sain. Le mélange actif selon l'invention peut être utilisé pour prévenir une infection en limitant la réplication virale intracellulaire et/ou en limitant la pénétration du virus dans la cellule.

Avantageusement, le mélange actif selon l'invention a une efficacité anti-réplicative préférentielle sur le métabolisme cellulaire de la cellule infectée (voies du stress oxydatif et des canaux calciques). Le mélange actif selon l'invention peut être utilisé spécifiquement pour lutter directement contre la réplication virale intracellulaire et contre une surinfection cellulaire par HSV-1 et/ou HSV-2 du même tissu infecté et/ou d'un tissu sain voisin du précédent.

Selon une variante, le mélange actif selon l'invention induit de façon hétérogène une inhibition de la production d'interleukine-6 (IL-6) d'un lambeau cutané humain et a ainsi une efficacité anti-inflammatoire.

Sur les figures :

La figure 1 représente les variations du potentiel rédox et de la concentration en $H_2O_2$ lors de la préparation du Mélange 1.

La figure 2 représente un abaque de Fe$^{3+}$ servant au dosage de l'efficacité rédox du Mélange 1.

La figure 3 est une représentation de l'efficacité rédox du Mélange 1 à différentes concentrations de celui-ci (absorbance de la quercétine par le Fe(III) en milieu plasmatique humain après contact (2 min.) avec le Mélange 1 (en

nM $MoO_4^{2-}$).

La figure 4 représente un abaque de l'efficacité rédox du Mélange 1 à différentes concentration de celui-ci.

La figure 5 représente l'influence du Mélange 1 à différentes concentration de $MoO_4^{2-}$ sur la production *in situ* en plasma humain de $Fe^{3+}$, i) issu du complexe transferrine-$Fe^{3+}$ après mise en contact avec la formulation, ii) sur l'équilibre $Fe^{2+}/Fe^{3+}$ et, iii) sur l'oxydation de la quercétine.

La figure 6 représente la production d'interleukine-6 (IL-6) dans les surnageants de culture de biopsies non-pathologiques de peau humaine stimulées (2 min.) par le Mélange 1 (24.3 $\mu$M de la substance active) ou NaCl (100mM) comme témoin. (D1-5: donneur 1 à 5, B1 ou 2: biopsie 1 ou 2).

La figure 7 représente la moyenne de la production à 6h et 24h post-contact d'interleukine-6 (IL-6) dans les surnageants de culture de biopsie non-pathologique de peau humaine stimulée (2 min.) par le Mélange 1 (24.3 $\mu$M de la substance active) ou NaCl (100mM) comme témoin.

La figure 8 est une représentation de l'efficacité rédox du Mélange 1 et du Mélange 2 à différentes concentrations (absorbance de la quercétine par le Fe(III) en milieu plasmatique humain après contact (2 min.) avec le Mélange 1 (en nM $MoO_4^{2-}$) ou avec le Mélange 2 ( en nM $La(MoO_4)^{1-}$).

## Exemples

**Exemple 1** - Exemple de mélange actif selon l'invention

Le présent exemple est réalisé avec un sel de molybdène. On prépare une composition de formule selon le tableau 1 (« Mélange 1 »), exprimée en concentration initiale des constituants :

Tableau 1

| Composants | Mélange selon l'invention |
|---|---|
| Molybdate de sodium | 20.7pM |
| BAPTA | 12.6$\mu$M |
| EGTA | 526$\mu$M |
| $H_2O_2$ | 353mM |
| $CH_3COOH/CH_3COONa$ | 70mM |
| pH (par NaOH) | 4.4 - 5.0 |
| $E^\circ_{rédox}$ | 300 à 420mV |

La composition est dite "initiale" car correspondant aux concentrations des réactifs ajoutés sans tenir compte du process catalytique mis en oeuvre.

Tous les constituants servant à la synthèse ainsi que le produit fini sont validés par spectre IR-FT.

Tableau 1bis

| Produit | Pureté | Formule brute | N° CAS |
|---|---|---|---|
| Molybdate de sodium dihydrate | 98-103% | $Na_2MoO_4.2H_2O$ | 10102-40-6 |
| BAPTA: (1,2-bis(o-aminophenoxy)ethane-*N,N,N',N'*-tetraacetic acid) | ≥98% | $C_{22}H_{24}N_2O_{10}$ | 85233-19-8 |
| EGTA: (ethylene glycol-bis(2-aminoethylether)-*N,N,N,N'*-tetraacetic acid) | ≥99% | $C_{14}H_{24}N_2O_{10}$ | 67-42-5 |
| Peroxyde d'hydrogène | 29-31% | $H_2O_2$ | 722-84-1 |
| Acide acétique glacial | 99.8-100.5% | $CH_3COOH$ | 64-19-7 |
| Acétate de sodium trihydrate | 99-101% | $C_2H_3NaO_2.3H_2O$ | 5010524 |
| Sodium hydroxyde | 10N | NaOH | 1310-73-2 |
| Eau déminéralisée | 1$\mu$S | $H_2O$ | 7732-18-5 |

Les données chiffrées des mesures effectuées durant et après la synthèse sont les moyennes issues de 3 productions.

Les quantités indiquées ci-dessous sont pour la préparation d'un litre de Mélange 1.

**A** - **ETAPE (i) : préparation de la solution tampon (solution ST)**

| | |
|---|---|
| CH$_3$COOH (100%; δ: 1.05g.cm$^{-3}$ (liquide, 20 °C)) | 4.0ml (70mM final) |
| CH$_3$COONa.3H$_2$O (100%) | 8g (59mM final) |
| Eau | qsp 1L |
| Vérification du pH : | 4.7-4.8 |

Température ambiante (20°C)

**B - ETAPE (ii) : préparation de la solution de complexe métallique (solution SC)**

| | |
|---|---|
| Na$_2$MoO$_4$.2H$_2$O (source de Mo(VI); 100%) | 200mg (2.4mM final) |
| Eau | qsp 340ml |

Agitation douce, température ambiante

**C** - **ETAPE(iii) : préparation de la solution initiale (Si1)**

| | |
|---|---|
| Eau | 900mL |
| CH$_3$COOH (100%) | 4mL (70mM) |

Ajouter très doucement et sous agitation très douce (250rpm),
pH : 2.9-3.8
E$_{rédox}$: 470-490mV
puis,

| | |
|---|---|
| H$_2$O$_2$ (30%) | qsp pour 1.2% final <u>en poids</u> et selon les dosages préliminaires |

<u>À 45 min.</u> :

pH : 2.70-2.90
E$_{rédox}$: 440-460mV
Par l'équation de Noble : [H$_2$O$_2$] = 380-410mM (Solution Si1)

**D** - **ETAPE (iv) : préparation de la solution initiale (Si2)**

| | |
|---|---|
| Introduire la "solution ST" dans la solution Si1 | 12mL (≈1mM acétate) |

Ajouter sous agitation douce.

À 45 min. :

pH : ≈ 2.90
$E_{rédox}$ : 460-480mV

Par l'équation de Noble : $[H_2O_2]$ = 360-410mM     (Solution Si2)

**E - ETAPE (v) : préparation de la solution peroxomolybdique S1**

Introduire la "solution SC" dans la solution Si2     10mL

À 45 min. :

pH : 2.80-3.00
$E_{rédox}$ : 450-480mV

Par l'équation de Noble : $[H_2O_2]$ = 370-400mM     (Solution S1)

**F - ETAPE (vi) : préparation de la solution S2**

Ajustement du pH sous agitation douce :

NaOH 9,9-10,1M     3.6 mL pour ≈ 0.9L du mélange 1

pH 4,5-5,0

À 45 min. :

pH : 4.60

$E_{rédox}$ : 380-390mV     (Solution S2)

**G - ETAPE (vii) : Ajout des agents chélatants - Préparation de la solution S4**

Introduction BAPTA (98.8%)     6mg/L,

après dissolution très lente du BAPTA sous agitation douce dans un aliquote de la formulation S2 (1.4L) préalablement équilibrée à 25°C (solution dite « S3.1 »).
Pool de la solution S3.1 avec la solution S2 pour former la solution S3.2.

À 45 min. :

pH : 4.40-4.70
$E_{rédox}$ : 380-390mV
Par l'équation de Noble : $[H_2O_2]$ = 370-390mM

Introduction EGTA (99.1%)        200mg/L dans la solution S3.2 pour former la solution S4

Dissoudre sous agitation douce.

À 45 min. :

pH : 4.40-4.60
$E_{rédox}$ : 380-390mV
Par l'équation de Noble : $[H_2O_2]$ = 370-420mM

**H** - **ETAPE (viii): ajustement du pH**

Ajustement du pH (NaOH 9,9-10,1M, c à d ≈400g/L) => pH : 4.60 ± 0.2

**I - ETAPE (ix)** : **ajustement du volume pour la solution finale (SF)**

Ajustement du volume par eau déminéralisée      qsp 1 litre

À 12-18 heures :

pH : 4.50-4.70
$E_{rédox}$ : 380-390mV
Par l'équation de Noble : $[H_2O_2]$ = 350-380mM
Par titrimétrie : $[H_2O_2]$ = 400-430mM
Densité : $\delta$ = 1.004g/ml

Le mélange selon l'invention, prêt à l'emploi, est stable à l'obscurité plus de 6 mois à température ambiante ou à 45°C.
L'hydro-peroxo-molybdate de Na est à une concentration de 24.3$\mu$M dans le Mélange 1 final.

**Exemple 2** - Signature par variation de potentiel rédox

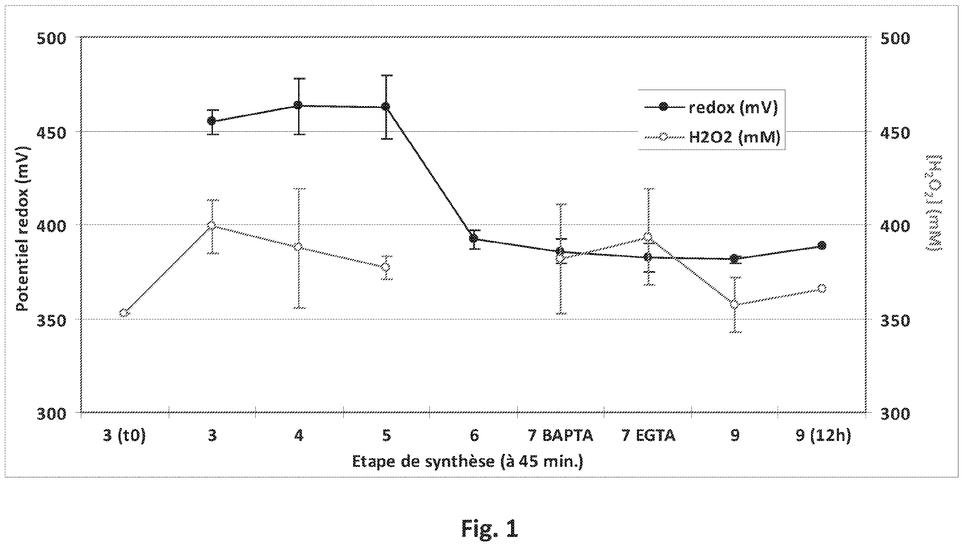

On évalue la signature du mélange actif selon la présente invention par la variation du potentiel rédox lors de la synthèse d'une composition préparée selon l'exemple 1.
D'après la Fig. 1, par rapport à la solution initiale (≈470mV; étape 3), diminution du potentiel rédox de ≈90mV (≈380mV) à l'étape 6 (ajustement du pH par la soude). Le potentiel rédox reste constant jusqu'à la fin de la synthèse (étape 9). Il en est de même lors du vieillissement des formulations du mélange de l'invention selon l'exemple 1 sur 6 mois à température ambiante (389mV ± 5mV) et à 45°C (389mV ± 8mV) pour un pH de 4,56 ± 0,40 et 4.53 ± 0.41, respectivement.

**Exemple 3** - Signature par consommation et production d'$H_2O_2$ - création de l'équilibre peroxo-réactif et du potentiel rédox du Mélange 1.

On évalue la signature du mélange actif selon la présente invention par consommation et production d'$H_2O_2$ lors de la synthèse d'une composition préparée selon l'exemple 1.
D'après la Fig. 1, par rapport à la concentration en $H_2O_2$ de solution initiale (353mM; étape 3 à t0), il est observée une brusque production peroxydique (≈42mM; dont une faible part d'acide peracétique) du fait de l'ajout de l'$H_2O_2$ dans la solution tampon (acide acétique/acétate). Si l'ajout d'$H_2O_2$ n'est pas réalisé dans une solution aqueuse préalablement tamponnée acide (étape 3; pH = 2.84 ± 0.1), il s'en suivrait sa dégradation spontanée et rapide du fait d'un pH (eau) trop élevé ($pKa_{HO2\cdot-/O2\cdot-}$: 4.8).

Il est observé à partir de l'étape 4 jusqu'à l'étape 7, une consommation limitée d'H₂O₂ (10mM ± 6mM), jusqu'à sa stabilisation à 381mM ± 5mM (étape 7; +BAPTA).

L'ajout de l'EGTA déplace l'équilibre peroxydique (réaction Fenton "like") par une dégradation peroxydique, c'est-à-dire de 393mM ± 29mM à l'étape 7, à 357mM ± 26mM à l'étape 9, et 365mM ± 15mM à l'étape 9 + 12 heures.

A l'étape 6, il est observé une chute brutale du potentiel rédox (de 463mV ± 15 mV à 392mV ± 17mV) par l'ajustement du pH. L'ajout (étape 7) des agents chélatants BAPTA puis EGTA contribue successivement à une stabilisation du potentiel rédox (386mV ± 5mV et 383mV ±6mV) par stabilisation des équilibres chimiques.

**Exemple 4** - Dosage de l'efficacité rédox du mélange de l'invention: méthodes quercétine

A - Les solutions réactives

1 - Quercétine

Une solution de quercétine dihydrate (2-(3,4-dihydroxyphenyl)-3,5,7-trihydroxy-4*H*-1-benzopyran-4-one dihydrate, 3,3',4',5,7-pentahydroxyflavone dihydrate; ($C_{15}H_{10}O_7.2H_2O$) à $10^{-3}$ mol/L est préparée en méthanol.

2 - Solution chlorhydrique

Une première solution d'acide chlorhydrique 1M est préparée en méthanol. Une seconde solution est préparée à 0.3 M en méthanol. La solution réactive est préparée en ajoutant 343µl de la solution 1M à 1357µl de la solution 0.3 M.

3 - Solutions de $Fe^{3+}$ (abaque de référence)

Une solution de $Fe^{3+}$ ($Fe_2(SO_4)_3.xH_2O$) à 2 mg/ml est préparée en eau HPLC. Elle est ensuite diluée pour obtenir huit solutions de référence à : 20, 100, 200, 300, 400, 500, 1000 et 1500 µg/ml. Ces solutions sont finalement diluées 20 fois en plasma humain ou en eau HPLC pour obtenir en final des concentrations de : 0, 1, 5, 10, 15, 20, 25, 50 et 75 µg/ml.

4 - Solutions de $Fe^{2+}$ (abaque de référence, contrôle d'activité)

Une solution de $Fe^{2+}$ ($FeSO_4.7H_2O$) à 89 mg/ml est préparée en eau HPLC. Elle est ensuite diluée pour obtenir trois solutions de référence à : 0.445, 4.45 et 44.5 mg/ml. Ces solutions sont finalement diluées 10 fois en eau HPLC pour obtenir en final des concentrations de : 0, 0.0445, 0.445 et 4.45 mg/ml.

B - Dosages par la méthode quercétine

La présence de $Fe^{3+}$ (abaque) ou sa production (abaque $Fe^{2+}$ et efficacité du mélange de l'invention après 2 min. de contact) sont quantifiées par la méthode quercétine.

Selon les conditions d'analyse ou d'utilisation du Mélange 1 :

« mélange$_{ox}$ » fait référence au mélange de selon l'invention à titre d'oxydant.
« mélange$_{réd}$ » fait référence au mélange de selon l'invention à titre de réducteur.

Les productions de $Fe^{3+}$ sont selon les équations:

$$\text{Transferrine-2Fe}^{3+} + \text{mélange}_{red} + 3H^+ \rightarrow \text{Transferrine}_{red} + 2Fe^{3+}_{(aq)} + \text{mélange}_{ox} \quad (1)$$

$$Fe^{3+}_{(aq)} + \text{mélange}_{red} + e^- \rightleftarrows Fe^{2+}_{(aq)} + \text{mélange}_{ox} \quad (2)$$

$$Fe^{3+}_{(aq)} + \text{quercétine}_{red} \rightarrow \text{quercétine}_{ox} + Fe^{2+}_{(aq)} \quad (3)$$

La réaction (1) est en milieu plasmatique.

La réaction (2) est en milieu plasmatique ou en eau HPLC et correspond à l'équilibre de la réaction de Fenton-Haber-Weiss.

La réaction (3) est la réaction d'oxydation de la quercétine pour évaluer le pouvoir rédox du Mélange 1.

En milieu acide et à 70°C, la quercétine est oxydée spécifiquement par le fer ferrique ($Fe^{3+}$; El Hajji et al 2006 et Balcerzak et al, 2008) porté par la transferrine en milieu plasmatique et non par le fer ferreux ($Fe^{2+}$). L'ion $Fe^{3+}$ est soluble dans ces conditions.

30µ! de la solution quercétine sont ajoutés à 170µ! de la solution réactive chlorhydrique. Après homogénéisation, 50µ! de l'échantillon ($Fe^{3+}$ en plasma humain ou en eau HPLC, $Fe^{2+}$ en eau HPLC, le mélange de l'invention après 2 min. de contact avec le plasma humain) sont ajoutés. Les solutions sont agitées fortement et brièvement puis incubée 1h à 70°C. Après incubation, les échantillons sont centrifugés 15 min. à 14.000 tpm et température ambiante. 100µl de surnageants sont prélevés. Le spectre UV de chaque échantillon est acquis de 230 à 500nm. Les blancs : eau, $Fe^{2+}$, $Fe^{3+}$, plasma sont soustraits selon le type d'expérimentation. Le pic d'absorption de la quercétine oxydée par le $Fe^{3+}$ produit à partir de la transferrine plasmatique et la réaction de Fenton-Haber-Weiss *in situ* est conservé (de 285-305 nm).

L'apex de la courbe est centré vers 292 nm quand l'expérience est réalisée dans de l'eau HPLC ou du plasma. Il peut se déplacer d'un maximum de 10 nm quand les blancs expérimentaux sont soustraits.

1 - Abaque $Fe^{3+}$

On réalise un abaque pour l'ion Fe(III) par exemple en mesurant l'absorbance en UV (sur la gamme 250-330nm) de la quercétine oxydée pour différentes concentrations de $Fe^{3+}$ (cf. la gamme $Fe^{3+}$ ci-dessus) en plasma humain, et en reportant la densité optique en fonction de la longueur d'onde.

A partir du spectre de la région 250-330nm, l'aire sous la courbe (AUC, pour Area Under the Curve) entre 285 et 305nm est calculée par la formule :

$$AUC\begin{smallmatrix}305nm\\285nm\end{smallmatrix} = \int_{285nm}^{305nm} f(x)dx = \int_{285nm}^{305nm} Absorbance \, . \, d(\lambda)$$

En conséquence, chaque aire de la surface du pic 285-305nm pour chaque point de la gamme de $Fe^{3+}$ est portée pour représentation graphique. La courbe de tendance est tracée (Excel), l'équation (le coefficient de corrélation $R^2$ dont la valeur la plus proche de 1 valide l'expérimentation) est déduite (Excel).

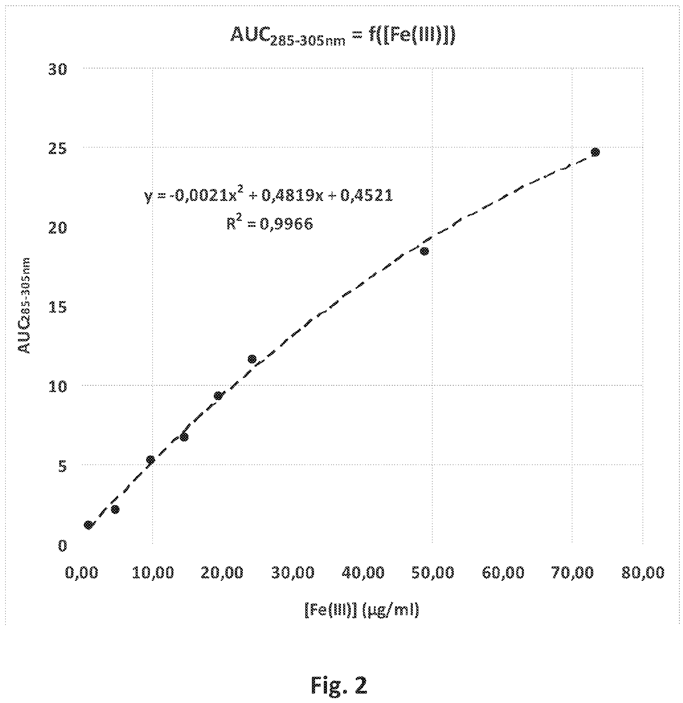

Elle peut être d'une forme linéaire ou polynomiale de type: $y = ax^2 + bx + c$. Par expérience cette relation peut être du type $y = -0,0021x^2 + 0,4819x + 0,4521$ ; $R^2 = 0,9966$ (Fig. 2). (4)

Pour un mélange selon l'invention, la concentration en équivalent $Fe^{3+}$ produit en milieu plasmatique ou à partir de $Fe^{2+}$ est le reflet du pouvoir oxydant de l'invention et est calculé à partir de l'équation (4). Il s'agit d'une méthode indirecte de mesure de l'efficacité rédox du Mélange 1 par dosage en UV de la quercétine oxydée par le $Fe^{3+}$. En conséquence il est évoqué une concentration en "équivalent" $Fe^{3+}$. De même, il est possible de comparer le pouvoir rédox d'une solution selon l'invention à tester, par rapport à une solution de référence selon l'invention, autrement dit, faire une comparaison avec quantification de l'efficacité rédox d'un mélange selon l'invention.

2 - Abaque $Fe^{2+}$

De la même manière que pour l'ion Fer(III), on réalise un abaque pour l'ion Fer(II). On mesure par exemple l'absorbance en UV (230-500nm) du pic quercétine oxydée par le $Fe^{3+}$ obtenu à partir du mélange de l'invention à 1.22µM de substance active (250 µg/L) et 2 min. de contact avec $Fe^{2+}$ pour la gamme décrite ci-dessus. Les mêmes analyses mathématiques en AUC telles que décrites pour l'ion Fer(III) peuvent être réalisées.

3 - Dosage de l'efficacité rédox du mélange de l'invention par exemple à 1.22 µM (250 µg/L) en substance active $MoO_4^{2-}$.

Selon le protocole décrit en B-, l'efficacité rédox d'un mélange selon l'invention (1.22 µM $MoO_4^{2-}$ en plasma humain) est testée pour: i) évaluer son vieillissement à température ambiante et à 45°C, ii) valider sa production et évaluer comparativement les formulations, iii) quantifier son passage transcutané, iv) sa biodisponibilité et, v) sa biophase.

Selon l'équation (4), on calcule l'aire spécifique du pic de l'absorbance en UV (en AUC) entre 285 et 305 nm du pic quercétine oxydée par le $Fe^{3+}$ libre produit après contact plasmatique 2 min. du mélange de l'invention (équations (1), (2) et (3)) à une concentration finale de 1.22 µM avec le complexe transferrine-$Fe^{3+}$. En moyenne, la production d'équivalent $Fe^{3+}$ ($Fe^{3+}_{eq}$) est de l'ordre de 30 à 50 µg/ml de plasma.

4 - Abaque d'estimation en milieu plasmatique de la concentration efficace du mélange selon l'invention

Le fer plasmatique n'est pas libre. Il est lié à la transferrine (ou sidérophiline) sous sa forme ferrique ($Fe^{3+}$) à raison de 1 à 2 résidus par molécule. Le fer sous sa forme ferreuse ($Fe^{2+}$) n'est pas circulant en dehors de sa complexation à l'hémoglobine. Il est considéré que le plasma humain utilisé n'est pas hémolysé. La réaction quercétine du mélange inventif avec le cuivre plasmatique est considérée comme négligeable.

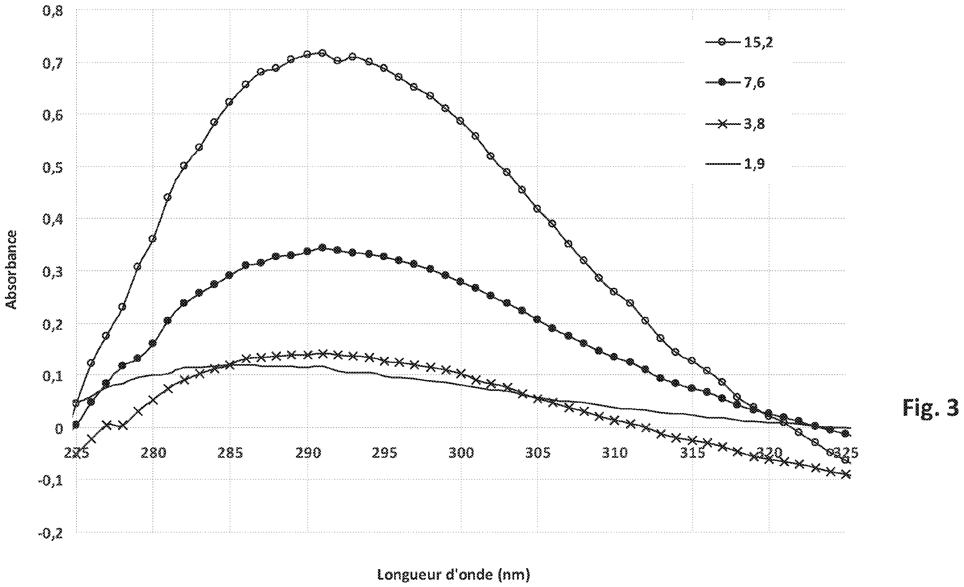

Une gamme de concentrations du mélange de l'invention (15.2, 7.6, 3.8 et 1.9 nM $MoO_4^{2-}$ final) a été incubée en plasma humain. La réaction avec la quercétine a été réalisée comme décrit en B-. Le pic d'absorption spécifique (soustraction du blanc plasma + quercétine) a été intégré comme décrit en B-1 (Fig. 3).

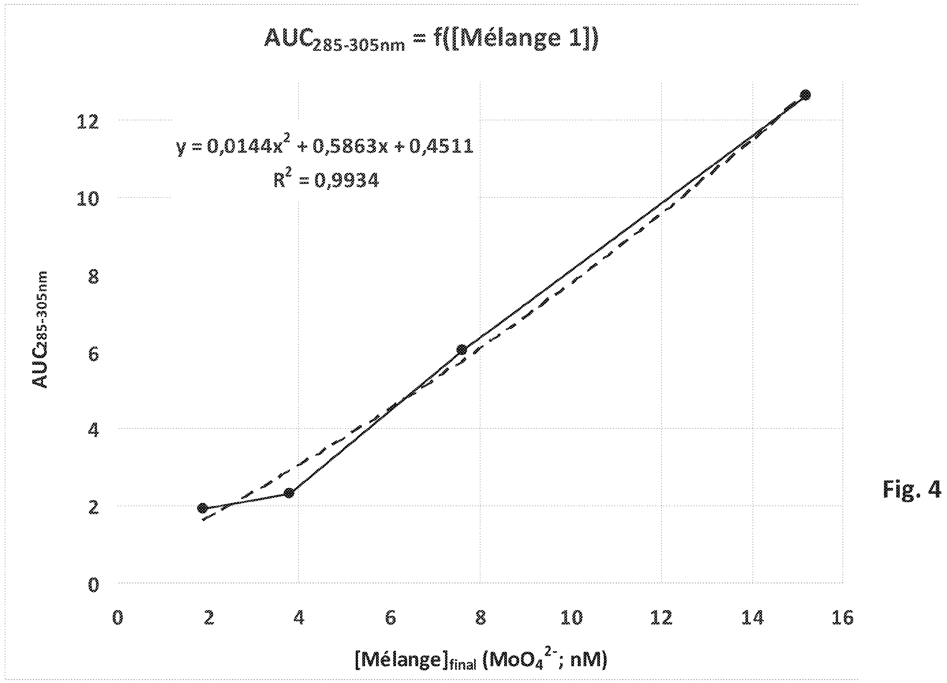

L'abaque a été tracé (Excel) en échelle polynomiale et l'équation de tendance calculée ($y = 0,0144x^2 + 0,5863x + 0,4511$ avec $R^2 = 0,9934$ (5); Fig. 4).

Une relation de proportionnalité (équation 5) est constatée entre la quantité de l'invention en concentration ajoutée au plasma humain et la quantité en équivalent de $Fe^{3+}$ (équation 4) détectée par la réaction d'oxydation de la quercétine, à savoir 28.95, 12.19, 3.97, 3.12 $\mu$g $Fe^{3+}_{eq}$/mL de plasma humain, selon la gamme du mélange de l'invention décrite ci-dessus.

**Exemple** 5 - Evaluation de l'efficacité oxydoréductrice de l'invention sur plasma humain

Après un ajout du mélange selon l'invention (exemple 1 - « Mélange 1 ») à du plasma humain et après un temps de contact de 2 min., jusqu'à une concentration de 0.03038 $\mu$M $MoO_4^{2-}$, la réaction d'oxydation de la quercétine par la production de $Fe^{3+}$ issu du complexe transferrine-$Fe^{3+}$ plasmatique et la réaction de Fenton-Haber-Weiss est optimale et linéaire (cf. exemple 4).

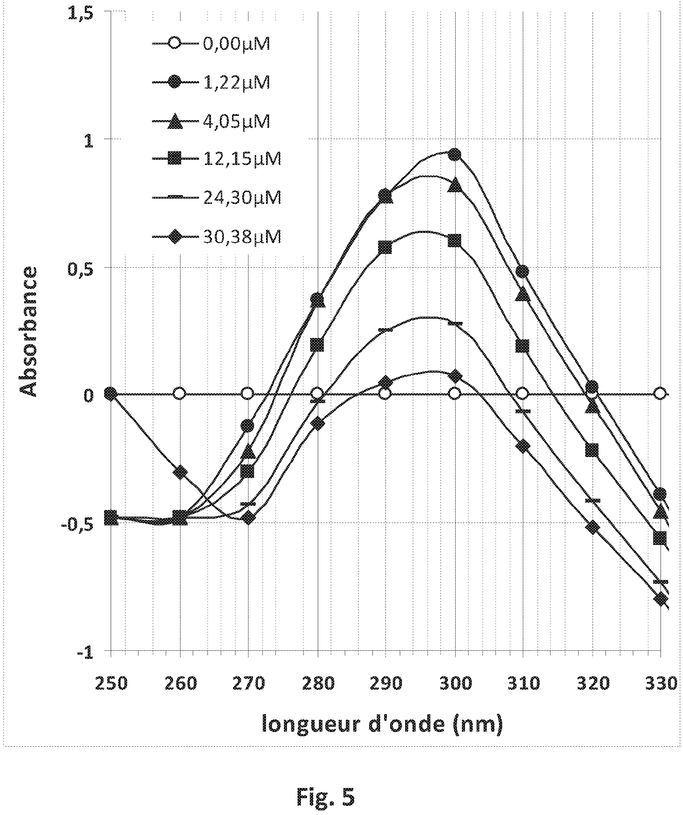

Après soustraction des blancs expérimentaux, une augmentation de la concentration de l'invention génère une diminution de la production *in situ* de quercétine oxydée par $Fe^{3+}$ du fait du déplacement des équilibres de la réaction de Fenton-Haber-Weiss ($Fe^{3+} \rightleftarrows Fe^{2+}$). Les concentrations finales de $MoO_4^{2-}$ testées étaient alors de 0.00, 1.22, 4.05, 12.15, 24.30 et 30.38 $\mu$M (Fig. 5).

**Exemple 6** - Efficacité anti-réplicative

A - Les modèles

- Quatre modèles de contact du mélange selon l'exemple 1 (« Mélange 1 ») ont été testés. Ils représentent les quatre possibilités physiologiques que le Mélange 1 peut rencontrer lors d'une application thérapeutique topique, à savoir :

  *Modèle 1* : Mélange 1 sur cellules infectées par HSV-1,
  *Modèle 2 :* Mélange 1 sur cellules non encore infectées par HSV-1,
  *Modèle 3 :* Mélange 1 sur cellules non encore infectées et Mélange 1 sur HSV-1 libre (non encore infectant),
  *Modèle 4* : Mélange 1 sur HSV-1 avant infection.

- Deux temps de contact du Mélange 1 ont été testés, à savoir 2 min. ou 1.5 min., puis élimination du Mélange 1.
- Deux concentrations du Mélange 1 ont été testées : 0,81 et 2,03 $\mu$M en substance active, c'est-à-dire 167 et 417 $\mu$g/L, respectivement.
- La méthode de quantification des efficacités est la qPCR (quantitative Polymerase Chain Reaction ; Mullis K. et al (1986) "Spécifie Enzymatic Amplification of DNA In Vitro: The Polymerase Chain Reaction", Cold Spring Harb. Symp. Quant. Biol., 51 (Pt 1), 263-273.)
- L'efficacité anti-réplicative (EA) du Mélange 1 sur HSV 1 a été évaluée en qPCR avec des primers spécifiques du génome viral (Kessler H.H. et al (2000) "Détection of Herpes simplex virus DNA by real-time PCR" J. Clin. Microbiol., 38(7), 2638-2642).
- La cytotoxicité du Mélange 1 (C) sur la lignée BHK-21 a été évaluée en qPCR avec des primers spécifiques du gène de la petite sous-unité ribosomale 18S (Texcell - Evry, France).
- La multiplicité d'infection (MOI) : 1, c'est-à-dire $5.10^5$ $TCID_{50}$ (Tissue Culture Infective Dose 50%).
- Les expériences de qPCR ont été réalisées à t0h (2h après contact entre BHK-21 et HSV-1, puis élimination d'HSV-1), t2h- , t4h- et t8h-post-infection.
- Le rapport EA/C nous indique l'index d'Efficacité *in vitro* (IE) du Mélange 1 correspondant à un modèle d'étude.

*Modèle 1* :

BHK-21 + HSV-1 (2h ; infection) => lavage (PBS) pour élimination virale => prélèvements t0h => + Mélange 1 (2 min.) => lavage (milieu de culture) pour élimination du Mélange 1 => incubation cellulaire 37°C => prélèvements t2h-, t4h- et t8h-post-infection.

*Modèle 2 :*

BHK-21 + Mélange 1 (1.5 et 2 min.) => lavage (PBS) pour élimination du Mélange 1 => cellules + HSV-1 (2h ; infection) => lavage (milieu de culture) pour élimination d'HSV-1 => prélèvements t0h => incubation cellulaire 37°C => prélèvements t2h-, t4h- et t8h-post-infection.

*Modèle 5 :*

BHK-21 + Mélange 1 (1.5 et 2 min.) => lavage (PBS) pour élimination du Mélange 1.
HSV-1 + Mélange 1 (1.5 et 2 min.) => lavage (PBS) pour élimination du Mélange 1.
Pool des cellules et des virus ayant été en contact préalable avec Mélange 1 (2h ; infection) => lavage (milieu de culture) pour élimination d'HSV-1 => prélèvements t0h => incubation cellulaire 37°C => prélèvements t2h-, t4h- et t8h-post-infection.

*Modèle 4 :*

HSV-1 + Mélange 1 (2 min.) => lavage (PBS) pour élimination du Mélange 1 => + BHK-21 (2h ; infection) => lavage (milieu de culture) pour élimination d'HSV-1 => prélèvements t0h => incubation cellulaire 37°C => prélèvements t2h-, t4h- et t8h-post-infection.

B - Résultats

Tableau 2 : Réduction à 8 heures de la multiplication d'HSV-1 dans les cellules BHK-21 après contact avec Mélange 1 (en µg/L de substance active) ou de l'aciclovir (ACV); Index d'Efficacité *in vitro* (IE).

| | Réduction réplication HSV-1 à t8h (%)* | | | | | IE à t8h / *modèle*** | | | | |
|---|---|---|---|---|---|---|---|---|---|---|
| Substance | Mélange 1 | ACV | Mélange 1 | | | Mélange 1 | ACV | Mélange 1 | | |
| Temps de contact (min.) | 2 | 2 | 2 | 1.5 | 1.5 | 2 | 2 | 2 | 1.5 | 1.5 |
| Concentration (µg/L) | 167 | 417 | $10^3$ | 167 | 417 | 167 | 417 | $10^3$ | 167 | 417 |
| *Modèle 1* | -97 | -98 | -12 | NT | NT | 37 | 48 | 2 | NT | NT |
| *Modèle 2* | -72 | -96 | -1 | -70 | -86 | 4 | 23 | 1 | 3 | 7 |
| *Modèle 3* | -93 | -97 | -6 | -78 | -90 | 14 | 35 | 1 | 4 | 10 |
| *Modèle 4* | -56 | -69 | -52# | NT | NT | 2 | 3 | 2# | NT | NT |

* La réduction de la réplication d'HSV (RR) par les substances testées (Mélange 1, aciclovir témoin) est exprimée en pourcentage et correspond au rapport de quantifications qPCR des génomes HSV-1 et de la cytotoxicité laquelle est évaluée par quantification de la sous-unité 18S de la cellule hôte: $RR = \{(100 \times ([HSV\text{-}1]_{test}/[18S]_{test})) / ([HSV\text{-}1]_{témoin+}/[18S]_{témoin+})\} - 100$.

** L'Index d'Efficacité anti-réplicative *in vitro* (IE) des substances testées est représenté par le rapport ([HSV-1] / [18S] évalué en qPCR spécifiques) du contrôle positif infecté par HSV ($[HSV\text{-}1]_{témoin+}/[18S]_{témoin+}$) vs. celui des substances testées (Mélange 1, aciclovir témoin) : $IE = ([HSV\text{-}1]_{témoin+}/[18S]_{témoin+}) / ([HSV\text{-}1]_{test}/[18S]_{test})$.

# [ACV]: 1 g/L

- Les meilleurs IE (Tab. 2) sont obtenus après 2 minutes de contact du mélange 1 et une concentration à 417 µg/L. Nous notons une influence significative du temps de contact sur les IE (1.5 min. et 2 min.; *modèles 2* et *3*).

Les IE obtenus avec les *modèles 1* et *3* avec 2 min. de contact et une concentration du mélange 1 de 167 µg/L, sont particulièrement significatifs.

- Quel que soit le modèle, les IE obtenus avec l'aciclovir (« ACV ») ne sont pas significatifs (Tab. 2).

<u>Tableau 3</u> : Pourcentages en quantification par qPCR (gène 18S ou HSV; à 8h post contact de 2 min.) : i) de la multiplication de la lignée BHK-21 (témoin: lignée non-infectée) et, ii) de la réplication de HSV-1 (témoin: lignée infectée).

| Gène/génome | 18S (%) | | | | | | HSV (%) | | |
|---|---|---|---|---|---|---|---|---|---|
| Témoin | Lignée + (PBS – HSV) | | | Lignée + (PBS + HSV) | | | | | |
| Substance test | Mélange 1 | | ACV (mg/L) | Mélange 1 | | ACV (mg/L) | Mélange 1 | | ACV (mg/L) |
| [substance active] | 167 µg/L | 417 µg/L | | 167 µg/L | 417 µg/L | | 167 µg/L | 417 µg/L | |
| *Modèle 1* | 45 | 27 | 108 (1) | 49 | 30 | 118 (1) | 1 | < 1 | 76 (1) |
| *Modèle 2* | 62 | 41 | 57 (1) | 68 | 44 | 62 (1) | 19 | 2 | 61 (1) |
| *Modèle 3* | 118 | 70 | 111 (1) | 98 | 58 | 92 (1) | 7 | 2 | 86 (1) |
| *Modèle 4* | 120 | 107 | 97 ($10^3$) | 139 | 123 | 111 | 64 | 38 | 54 ($10^3$) |
| Tests | 1 | 2 | 3 | 4 | 5 | 6 | 7 | 8 | 9 |

- Pour le Mélange 1 (à 167 à 417 µg/L) ou l'ACV (à 1 ou $10^3$ mg/L), quelque soit le témoin utilisé (lignée + PBS - HSV ou lignée + PBS + HSV), les résultats de quantification de la 18S sont comparables pour un même modèle (Tab. 3, test 1 *vs.* 4, 2 *vs.* 5, 3 *vs.* 6).

- Pour le Mélange 1, une efficacité antiréplicative remarquable dose dépendante (Tab. 3, tests 7 et 8) est constatée pour les modèles 1, 2 et 3. Ceci n'est pas observé pour ACV (Tab. 3, test 9).

- Une cytotoxicité comparable du Mélange 1 (167 µg/L) et de l'ACV (1mg/L) (Tab. 3) est notée selon les *modèles* 2 et 3 (Tab. 3, tests 1, 3, 4 et 6).

- Une absence d'efficacité anti-HSV (Tab. 2 et Tab. 3, test 9) pour l'ACV (1 mg/L ou 1g/L) est notée. Les modèles introduits pour l'étude du Mélange 1 peuvent ne pas correspondre à une étude portant sur l'ACV.

Pour le *modèle 1* (Mélange 1 sur cellules infectées):

- Le meilleur IE (Tab. 2) avec les deux concentrations du mélange 1 (2 min. de contact, 167 et 417 µg/L, IE : 37 et 48, respectivement).
- Le Mélange 1 a une efficacité directe (Tab. 2 et 3) sur la réplication virale intranucléaire, sur le métabolisme cellulaire probablement.
- Le Mélange 1 a une bonne spécificité de la cellule infectée (Tab. 3, tests 4 et 5; modèle 1 *vs.* 2).

Pour le *modèle* 2 (Mélange 1 sur cellule avant l'infection):

- Un IE intéressant (Tab. 2; 2 min. de contact et 417 µg/L, IE : 23).
- Le Mélange 1 a une action sur la cellule laquelle devient moins réceptrice à l'infection et/ou acquiert un métabolisme incompatible avec la réplication du virus (Tab. 3, tests 7 et 8).

Pour le *modèle 3* (Mélange 1 sur : cellule non infectée et HSV-1, séparément et avant pool):

- Un IE intéressant (Tab. 2) avec les deux concentrations du mélange 1 (2 min. de contact, 167 et 417 µg/L, IE : 14 et 35, respectivement).
- Selon la dose appliquée (167 ou 417 µg/L) une cytotoxicité nulle (Tab. 3, test 4) ou limitée (Tab. 3, test 5) pour une très bonne efficacité antivirale (Tab. 3, tests 7 et 8, respectivement).
- Nous avons une efficacité du mélange 1 qui serait un cumul du *modèle 2* avec une action importante sur la cellule et du *modèle* 4 avec une action limitée sur le virus seul (Tab. 2 et 3).

Pour le *modèle 4* (Mélange 1 sur HSV-1):

- Un IE faible (2 min. de contact, 167 et 417 µg/L, IE : 2 et 3, respectivement; Tab. 2) qui est confirmé par une absence de cytotoxicité mais une réplication virale importante (Tab. 3, tests 8 et 9).

C - Conclusions

Sur la lignée BHK-21, les principales efficacités du Mélange 1 est : i) d'avoir une cytotoxicité préférentielle de la cellule infectée, ii) d'inhiber la réplication virale dans la cellule infectée et, iii) de limiter l'infection de la cellule saine.
La cible peut être membranaire (récepteurs au virus, par exemple) et/ou métabolique (régulation des voies du stress et apoptotique, par exemple).

La reconnaissance et l'internalisation virale

D'après le *modèle 4,* le Mélange 1 a une faible efficacité anti-réplicative directe au niveau du virus. Dans ces conditions expérimentales, proches de l'utilisation topique thérapeutique, cette observation est en faveur d'une altération limitée des récepteurs viraux à la cellule : gB (HSV-2), gC (HSV-1), gD et gH/gL (HSV-1 et -2). Le Mélange 1 n'altèrerait que de façon limitée les glycoprotéines virales par sa réaction de Fenton-Haber-Weiss *in vitro.*
Au même titre, nous ne notons pas d'influence importante du Mélange 1 sur la membrane phospholipidique de l'HSV qui pourrait perturber la fusion membranaire et la pénétration.
Dans ces conditions expérimentales, le Mélange 1 n'est pas un virucide.

La réplication virale et le métabolisme cellulaire :

La technique de qPCR utilise le lysat cellulaire à 8h post-infection. Une diminution de la réplication virale peut résulter d'une mauvaise reconnaissance et/ou internalisation et/ou réplication (modification du métabolisme cellulaire induit par l'infection et/ou le Mélange 1).
Si la voie de relargage viral était altérée par le Mélange 1 sans que les autres voies le soit et quel que soit le modèle, les IE résultants seraient beaucoup plus faibles du fait de l'accumulation cytoplasmique des virions.
D'après le *modèle 2* (IE : 23 à 2 min. de contact et 417 µg/L), une faible réplication virale après un contact cellulaire préalable avec le Mélange 1 (Tab. 2 et Tab. 3, test 8) est observée.
D'après ce modèle, la cellule BHK-21 a été en contact 2 min. avec Mélange 1. Le temps de contact avec HSV en milieu de culture complet et nutritif a été de 2 heures. Le Mélange 1 a induit des modifications cellulaires ayant une durée de vie perdurant au-delà de 2 heures et laissant présager une efficacité prophylactique de Mélange 1.
Outre que la reconnaissance d'HSV-1 par la cellule étant pour part affectée par le Mélange 1 du fait de son action probable et limitée sur la glycoprotéine virale gD (38% de réplication; Tab. 2), cette reconnaissance peut aussi être affectée par une action sur les récepteurs cellulaires à gD (HVEM et nectine) ainsi que sur les deux autres récepteurs cellulaires de surface à gB et gH/gL que sont les héparanes sulfates et les intégrines.
Du fait de la perméation radicalaire (modification du potentiel rédox intracellulaire et/ou transmembranaire, régulation des voies du stress oxydatif), les influx calciques cellulaires sont mobilisables. Ces influx calciques cellulaires sont de même induits par l'infection HSV. Cependant ceux-ci sont démobilisables immédiatement du fait de l'introduction dans le Mélange 1 d'agent chélatants libres et non perméants (limitation de la cytotoxicité cellulaire). D'autres modifications cellulaires majeures en revanche ne sont pas à exclure et pourraient correspondre à des oxydations ou réductions protéiques (ponts cystéinyls, par ex.) et en particulier de protéines structurales pouvant intervenir dans l'endocytose virale.
D'après le *modèle 3* (Tab. 2 et Tab. 3, tests 7 et 8), il est observé une diminution importante de la réplication virale (IE : 14 et 35 à 2min. de contact, 167 et 417 μg/L, respectivement).

Deux phénomènes se cumulent. Le premier est une action limitée au niveau du virus (cf. *modèle 4)*, le second, plus important (cf. *modèle 2*) est au niveau de la cellule.

L'addition des IE des *modèles 2* et *4* (2 min. et 417 μg/L), à savoir 23 et 3, respectivement, donne une somme (26) inférieure mais proche à celle expérimentalement obtenue pour le *modèle 3,* à savoir 35.

Il est envisagé un cumul potentialisé i) d'une mauvaise reconnaissance des partenaires HSV et cellule et ii) une internalisation et/ou une réplication inhibée notamment par altération du métabolisme cellulaire.

Le *modèle 1* (IE : 48 à 2 min. de contact et 417 μg/L) est le plus performant.

L'action du Mélange 1 anti-infectieuse est préférentielle sur la cellule que sur le virus. Cette action est d'autant plus performante que la cellule est préalablement infectée.

Dans ce modèle qui correspond à la meilleure problématique thérapeutique, il est évident que le Mélange 1 a une efficacité pharmacologique intracellulaire majeure spécifique. Pour 417 μg/L de substance active, le tableau 3 montre que la quantité de sous-unités ribosomales 18S est inférieure (selon le témoin utilisé, 27 et 30%, tests 2 et 5) dans ce modèle que dans le *modèle 2* (selon le témoin utilisé, 41 et 44%, tests 2 et 5) et le modèle 3 (selon le témoin utilisé, 70 et 58%, tests 2 et 5) qui est un argument de spécificité de la cellule infectée *vs.* la cellule saine.

Les cibles du Mélange 1 dans ce modèle ne peuvent être ni la reconnaissance ni l'internalisation, mais bien des modifications métaboliques. Celles-ci sont placées en un nouvel équilibre, notamment, du fait du passage radicalaire transmembranaire, la modification du potentiel rédox de la cellule et la présence des agents chélatants extérieurs, entre les voies du stress oxydatif (déjà induit par l'infection), les flux calciques (déjà induit par l'infection), l'inhibition du relargage calcium-dépendant des virions, les modifications par oxydation de protéines impliquées directement ou indirectement dans la réplication virale.

**Exemple 7** - Évaluation *ex vivo* de la production d'interleukines

Des biopsies (8 biopsies et 4 donneurs) de peau humaine non pathologiques issues d'actes chirurgicaux pour réduction de poids ont été traitées *ex vivo* par le Mélange 1 (5 mg/L en substance active; 2 min.). Les surnageants dans lesquels les biopsies se trouvaient ont été prélevés à 6h et 24h post-contact afin de quantifier les interférons-α (IFN-α; inhibition de l'infection par l'HSV; Mikloska Z. et al (2001) "Alpha and Gamma Interferons Inhibit Herpes Simplex Virus Type 1 Infection and Spread in Epidermal Cells after Axonal Transmission", J. Virol., 75(23), 11821-11826*),* -β (IFN-β; inhibition de la réplication d'HSV; Sainz Jr. B. et al (2002) "AlphalBeta Interferon and Gamma Interferon Synergize To Inhibit the Réplication of Herpes Simplex Virus Type 1", J. Virol., 76(22), 11541-11550*)* et l'interleukine 6 (IL-6; marqueur de l'inflammation).

Le contrôle expérimental positif est un soluté physiologique (NaCl 100mM).

Aucune modification du taux basal cellulaire d'IFN-α et d'IFN-β n'a été notée.

Selon les biopsies et les donneurs et en conséquence du traumatisme de l'acte chirurgical et de la manipulation expérimentale:

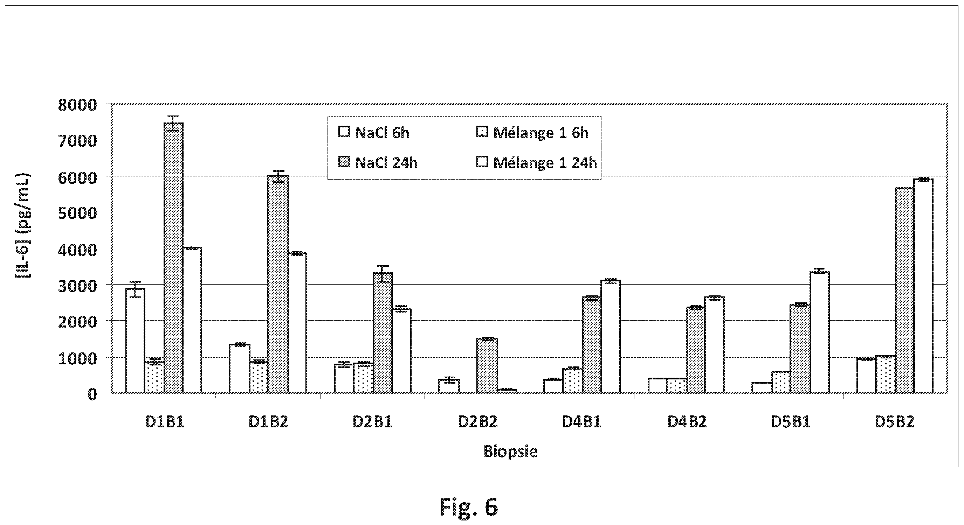

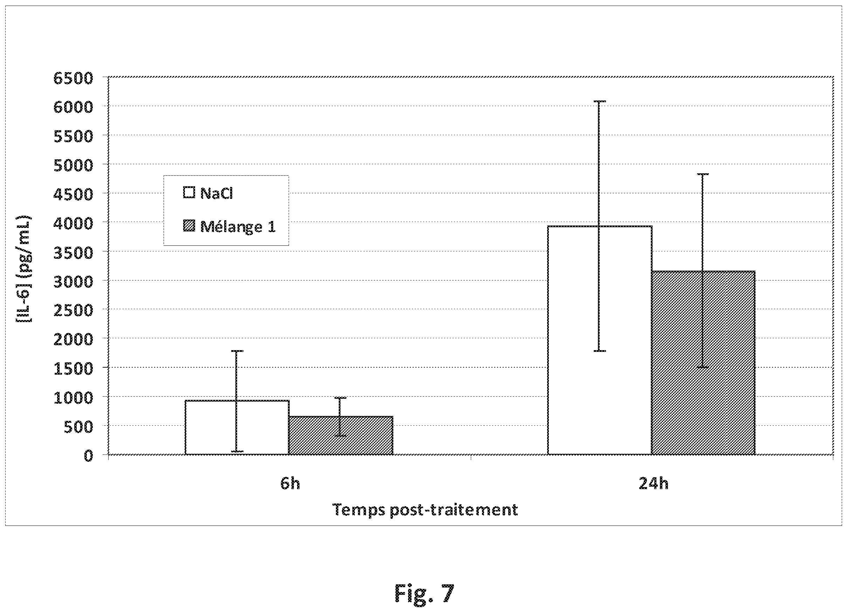

À 6h post-contact (Fig. 6 et 7):

- avec NaCl témoin, production d'IL-6 basal, de 277 pg/mL ± 11 à 2866 pg/mL ± 206.
- avec Mélange 1, inhibition de la production d'IL-6 par rapport au témoin NaCl, de 0 pg/mL à 1015 pg/mL ± 17.

À 24h post-contact (Fig. 6 et 7):

- avec NaCl témoin, production d'IL-6 basal, de 1485 pg/mL ± 37 à 7454 pg/mL ± 199.
- avec Mélange 1, inhibition de la production d'IL-6 par rapport au témoin NaCl, de 84 pg/mL ± 15 à 5910 pg/mL ± 29.

Conclusions:

- une hétérogénéité de production et surproduction (6h et 24h, respectivement) d'IL-6 post-contact NaCl et Mélange 1 (Fig. 6).
- une inhibition hétérogène de production d'IL-6 post-contact avec Mélange 1 (5 mg/L en substance active) qui est en moyenne de 28% à 6h et de 19% à 24h, par rapport au témoin NaCl (Fig. 6).

Par cette inhibition de production, Mélange 1 a la propriété d'être un anti-inflammatoire (Fig. 7).

**Exemple 8** - Dégradation plasmatique et ½ vie

Un des donneurs de radicaux (principalement HO$_2$$^•$, lequel a une durée de vie jusqu'à quelques secondes et un indice de pénétration cellulaire notable) par réaction de type Fenton-Haber-Weiss de la substance active MoO$_4$$^{2-}$ du Mélange 1 est H$_2$O$_2$. La dégradation de ce traceur a été suivie pour évaluer la vitesse de consommation du Mélange 1 ou sa désactivation.

Technique :

Le Mélange 1 (68.9 $\mu$M ou 14.2 $\mu$g/L final) a été incubé dans du plasma frais humain. La cinétique de disparition d'H$_2$O$_2$ a été suivie par réaction peroxydasique sur des aliquotes prélevés à des temps successifs.

Résultats :

- En moyenne (n =10), le pourcentage de dégradation de la substance traceur du Mélange 1 en plasma humain à température ambiante après 2 min. de contact est de 85.3 $\pm$ 9.4%, dont 74% $\pm$ 15.2 % dans la première minute.
- Dans la première minute, le *"Dégradation Time 50%"* ou DT$_{50}$ = 0.812min. pour une solution du Mélange 1 à 68.9 $\mu$M initial en substance active et 1mM H$_2$O$_2$ initial.
- La vitesse de dégradation K$_{cH2O2}$ : $\approx$ 740 $\mu$moles du Mélange 1 à 1mM initial en H$_2$O$_2$ / min. / L de plasma ou K$_{cMoO42-}$ : $\approx$ 51.0 $\mu$moles du Mélange 1 à 68.9 $\mu$M initial en substance active MoO$_4$$^{2-}$ / min. / L de plasma, dans la première minute d'incubation à température ambiante.
- Pour un échantillon de 200$\mu$L du Mélange 1 à 24.3 $\mu$M (5.0 mg/L) de substance active ou 353 mM (12 g/L) du traceur H$_2$O$_2$, dans le cadre d'une application thérapeutique topique, il y a $\approx$ 5 nmoles de substance active MoO$_4$$^{2-}$ ou 70 $\mu$moles du traceur H$_2$O$_2$. Cet échantillon, dans 1L de plasma, est dégradé en $\approx$ 0.1 minute.

**Exemple 9** - Hématotoxicité limitée sur le sang périphérique

L'objectif de cette étude est d'évaluer l'hématotoxicité du Mélange 1 (24.3 $\mu$M ou 5.0 mg/L final) après incubation 2, 3 et 5 minutes dans du sang périphérique humain. La toxicité dont l'hématolyse est évaluée en suivant les principaux paramètres sanguins, à savoir les numérations des hématies, des leucocytes, l'hématocrite, des plaquettes, le volume globulaire moyen et le taux d'hémoglobine.

Conclusions :

Les paramètres hématologiques concernant les numérations des hématies, des leucocytes, l'hématocrite, le volume globulaire moyen et le taux d'hémoglobine ne sont pas modifiés par une incubation jusqu'à 5 min. avec le mélange selon l'invention.

La numération plaquettaire est modifiée par le contact avec le Mélange 1 de -35% immédiatement après dès son ajout, à -69% à 5 minutes.

**Exemple 10** - Passage transcutané limité du mélange selon l'invention, absorption, biophase et biodisponibité (OCDE 428; EMEA, Human Guideline, 2001).

Le Mélange 1 utilisé était cinq fois plus concentré (Mélange 1x5) de façon à mettre en évidence les plus faibles passages de substances. L'objectif de cette étude était d'évaluer (référence t0, de t2min., à t60min.) le passage du Mélange 1x5 ainsi que celle du traceur H$_2$O$_2$ au travers de biopsies cutanées humaines non pathologiques.

Ce protocole est appliqué à trois types de substrat : biopsie totale, biopsie disséquée en épiderme d'une part et derme d'autre part.

Technique :

12 biopsies issues de 2 donneurs ont été testées (mesures dans la goutte déposée sur le lambeau [30$\mu$L]) et dans le milieu en dessous de celui-ci [800$\mu$l]) selon deux méthodes (Tab. 4) : i) la quercétine qui évalue l'efficacité rédox du Mélange 1×5 et, ii) la peroxydase qui mesure la concentration en traceur H$_2$O$_2$.

La goutte (30 $\mu$L) déposée au-dessus des biopsies et des lambeaux épidermiques et dermiques est 5 fois plus concentrée en substance active (121.5 $\mu$M ou 25.02 mg/L) et en traceur (1.77 M ou 60 g/L) que la formulation théra-

peutique (24.3 μM ou 5.0 mg/L et 352.8 mM ou 12 g/L, respectivement), Dans les 30 μL du dépôt de Mélange 1x5, il y a 3.65 nmoles ou 750 ng de substance active et 53 μmoles ou 1.8 mg de traceur.

Résultats :

Tableau 4

|  | Test | Essai | Biopsie* | Épiderme* | Derme* |
|---|---|---|---|---|---|
| Goutte | 1 | Efficacité rédox | -1.4 à -10.5 | -5.7 à -23.9 | NT |
|  | 2 | Traceur H$_2$O$_2$ | -16.5 à -57.7 | -72.5 à -99.9 | -99,6 |
| Milieu | 3 | Efficacité rédox | +3,7 à +9,0 | +0,8 à +80,6 | +44,5 à + 100 |
|  | 4 | Traceur H$_2$O$_2$ | < +0.001 ** à +0.09 | +0.38 à +1.7 | +0.16 à +0.46 |
| * en % par rapport à t0 (-: diminution, +: augmentation) ** limite de détection. | | | | | |

Une diminution faible à modérée (test 1) de l'efficacité rédox du Mélange 1 est observée dans la goutte au dessus du lambeau (de -1.4 à -23.9%).

Une diminution modérée à importante (test 2) de la quantité du traceur H$_2$O$_2$ du Mélange 1 est observée dans la goutte au dessus du lambeau (de -16.5 à -99.9%).

Une augmentation faible à importante (test 3) de l'efficacité rédox du milieu sous le lambeau est observée. Elle est fonction du lambeau et de son type. Quand il s'agit de la biopsie complète, cette augmentation due au passage transcutané est faible. Elle est en moyenne de 6.35% et correspond au passage de 232 pmoles (47.6 ng) de substance active du Mélange 1. Quand il s'agit de l'épiderme ou du derme, cette augmentation est très variable (de 0.8 à 100%). Ceci est très vraisemblablement du aux différences de structures histologiques (pores, irrigation sanguine dermique, terminaisons nerveuses épidermiques, par exemple).

Une augmentation non-détectable à faible (test 4) de la quantité du traceur H$_2$O$_2$ dans le milieu sous le lambeau est observée quel que soit son type (de < +0.001 à 1.7%). Quand il s'agit de la biopsie complète, cette augmentation due au passage transcutané est particulièrement faible (< 0,53 à 47,7 nmoles, c'est -à-dire < 0,018 à 1,62 μg). Quand il s'agit de l'épiderme ou du derme, en moyenne cette augmentation est faible (1.04 et 0.31%, c'est-à-dire 0.55 μmoles et 0.16 μmoles, ou 18.7 μg et 5.6μg, respectivement).

Conclusions :

Il y a indépendance (absorption, désactivation, perméation) tissulaire des deux paramètres considérés typiques du Mélange 1 que sont l'efficacité rédox due à la substance active et le dosage du traceur peroxydique donneur de radicaux.

En considérant la biopsie totale, l'indice de pénétration du Mélange 1 correspond à l'épaisseur du lambeau à savoir de 1 mm (épaisseur moyenne d'un épiderme) à 2 mm.

L'épiderme contient de nombreuses terminaisons nerveuses pouvant être le siège de relargages axonaux de virions herpétiques chez le patient. Elles sont les cibles du Mélange 1 au même titre que les cellules épidermiques infectées ou avant une infection potentielle. En considérant la succession des deux tissus, la biophase du Mélange 1 est quasi strictement celle de l'épaisseur de l'épiderme (1 mm) et est atteinte en totalité pour une absorption tissulaire cible maximale.

En considérant que le derme seul soit vascularisé, au maximum 80.6% de l'efficacité rédox du Mélange 1 peut se retrouver à son contact et 1.7% du traceur. Dans ces conditions expérimentales, la biodisponibilité du Mélange 1×5 est de 2.94 nmoles ou 605 ng de la substance active et 0.9 μmole ou 30.6 μg de traceur peroxydique.

En étude de génotoxicité *in vitro,* par le test d'Ames, le mélange selon l'invention et ses métabolites n'est pas mutagène en présence de l'activateur S9 (5/5 souches de *Salmonella*)*,* il est mutagène sur 1/5 souche en son absence.

En application locale sur peau stimulée ou non, le mélange selon l'invention (24.3 μM ou 5.0 mg/L en substance active) n'a pas de toxicité d'irritation associée.

En étude d'irritation buccale et vaginale, le mélange selon l'invention (24.3 μM ou 5.0 mg/L) n'est pas irritant (score 0/16) et très faiblement (score 1/16), respectivement.

En étude très sensible d'irritation de la membrane chorioallantoïque d'oeuf de poule, le Mélange 1 (18.23 et

24.3 $\mu$M ou 3.75 et 5.0 mg/L) est modérément irritant (Index d'irritation ou IS = 6.8 $\pm$ 0.4 et 6.3 $\pm$ 0/21, respectivement).

En étude de toxicité aigüe par application dermique, le mélange selon l'invention aux doses de 25.0 et 31.3 $\mu$g/kg de substance active contenus dans 60 et 75 mg/kg de traceur $H_2O_2$, respectivement, est bien toléré sans symptômes, pour une Dose Maximale Tolérée (DMT) et une "Dose Maximale Sans Effet Néfaste Observable" (DMSENO) ou "No Observed Adverse Effect Level" (NOAEL) de 31.3 $\mu$g/kg de substance active et 75 mg/kg de traceur.

En étude de toxicité aigüe par injection intraveineuse, le mélange selon l'invention aux doses de 10.4 et 12.5 $\mu$g/kg de substance active contenue dans 25 et 30 mg/kg de traceur $H_2O_2$, respectivement, est toléré sans symptômes notables pour une "Dose Maximale Tolérée" (DMT) de 12.5 $\mu$g/kg de substance active.

**Exemple 11** -Toxicité acceptable du mélange selon l'invention

**I**- **Mutagénicité** *in vitro* **(test d'Ames** - **OCDE 471)**

L'objectif de cette étude a été d'évaluer l'activité mutagène du mélange actif selon l'invention (Mélange 1) et de ses métabolites (produits par la fraction S9 de foie de rat) sur les souches de *Salmonella typhimurium* TA97a, TA98, TA100, TA102 et TA1535.

Conclusions :

- Sans système d'activation S9, le mélange actif (7.7 $\mu$M ou 2.43 $\mu$M c'est-à-dire 1.58 mg/L ou 0.50 mg/L) selon l'invention et ses métabolites n'ont pas d'effets mutagènes sur les lignées TA97a, TA98, TA100 et TA1535.
- Avec système d'activation S9, le mélange actif (Mélange 1$\times$5 : 121.5 $\mu$M ou 25.02 mg/L) selon l'invention et ses métabolites n'ont pas d'effets mutagènes sur les lignées TA97a, TA98, TA100, TA102 et TA1535.
- Sans le système d'activation S9, le mélange actif selon l'invention n'est pas mutagène sur la souche TA102 à 0.50 mg/L

Dans ce test *in vitro,* l'effet mutagène du mélange actif et de ses métabolites selon l'invention est limité pour ses utilisations (souche TA102).

**II** - **Dermato sensibilisation après induction (femelle Hamster: OCDE 406, ISO 10993-10: 2013 et ICH Mémorandum SCCP 2005)**

Selon le protocole de Magnusson & Kligman *(*Magnusson B. et al (1969) "The identification of contact allergens by animal assay. The guinea pig maximisation test", J. Invest. Dermatol., 52(3), 268-276*),* les potentialités de sensibilisation de la peau de femelles de hamster ont été étudiées selon trois protocoles.

Protocole 1 : Détermination de la dose limite tolérée

Des concentrations du Mélange 1 de 1.25 mg/L à 20.00 mg/L de substance active contenue dans 3g/L à 48 g/L de traceur $H_2O_2$, respectivement, en "patch" de 1 ml ont été appliquées 24h sur le flanc des animaux, puis les animaux ont été observés.

La concentration du Mélange 1 à 10 mg/L de substance active contenue dans 24 g/L du traceur est bien tolérée et cause une irritation modérée.

La concentration du Mélange 1 à 5.0 mg/L de substance active contenue dans 12 g/L de traceur $H_2O_2$ est bien tolérée et est considérée comme la dose maximale non irritante.

Protocole 2 : Détermination de la dose limite tolérée après induction par injection sous-cutanée.

Des concentrations de 5 mg/L à 10 mg/L de substance active contenue dans 12 g/L à 24 g/L du traceur $H_2O_2$, respectivement, du Mélange 1 ont été injectée (100 $\mu$L) par voie sous-cutanée. À 8 jours (premier challenge), une première application topique en "patch" de 1 ml a été réalisée. À 27 jours (second challenge), une seconde application topique en "patch" de 1 ml a été réalisée. Les observations ont été faites à 29 jours.

La concentration du Mélange 1 à 5 mg/L de substance active, contenue dans 12 g/L du traceur, est bien tolérée et ne produit pas d'irritation.

Protocole 3 : Etude de l'irritation induite par stimulation et application topique du Mélange 1 à 5 mg/L de substance active contenue dans 12g/L du traceur H₂O₂.

100µl de Mélange 1 à 5.0 mg/L a été injectée par voie sous-cutanée. À 4 jours, la zone injectée a été stimulée par une solution de sodium dodécyl sulfate (SDS) à 10% avant une application topique du Mélange 1 (1ml) en "patch". À 6 jours, une seconde application topique a été réalisée. À 21 jours (challenge) une application topique du Mélange 1 (1 ml) en "patch" avec des concentrations de 6.26 à 10 mg/L de substance active contenue dans 15 à 24 g/L de traceur a été réalisée.

Le Mélange 1 à 5.0 mg/L de substance active contenue dans 12g/L de traceur est bien toléré et le score de sensibilisation cutané par la formulation est égal à 0/3.

Conclusions : Le Mélange 1 à 5.0 mg/L de substance active n'est pas irritant. Aucune altération macroscopique de la peau des animaux n'a été observée. En conséquence, il n'y a pas eu nécessité d'étude anatomo-pathologique.

## III - **Irritation buccale et vaginale (lapine; OCDE(99)2Q, -21, -23 -24, -(95)115, - (02) 9 et ISO-10993-10: 2013)**

L'objectif de cette étude a été d'évaluer la tolérance vaginale et buccale du Mélange 1 à 5.0 mg/L de substance active contenue dans 12 g/L de traceur selon une exposition conforme à une utilisation thérapeutique humaine de 5 jours.

Du jour 0 au jour 6, chaque 24h, les animaux sont traités (1 ml) par voie buccale et vaginale. A jour 7, les animaux sont observés et leur poids mesuré.

Conclusions : La concentration du Mélange 1 à 5.0 mg/L de substance active est bien tolérée. L'index d'irritation buccale et vaginal est de 0/16 et 1/16, respectivement. En conséquence, il n'y a pas eu nécessité d'étude anatomo-pathologique.

## IV - **Test d'irritation oculaire (ICCVAM App B)**

Le test d'irritation oculaire utilisé a été le test de substitution HET-CAM (Hen's Egg Test - Chorioallantoic Membrane), publié au J. O. en date du 26/12/1996. Il est recommandé par l'ICCVAM.

Bien que cette méthode particulièrement sensible ne soit pas officiellement validée par l'U.E., elle est acceptée pour une utilisation visant à repérer les substances même légèrement irritantes dont celles qui seraient des irritants oculaires afin de leur attribuer l'étiquette R41 (2002).

Brièvement, la méthode consiste à appliquer une substance test du Mélange 1 à 3.75 et 5.00 mg/L de substance active dans 9 et 12 g/L de traceur, respectivement, sur la membrane chorioallantoïque d'un oeuf de poule fécondé. A 0.5, 2 et 5 min. post-contact, les trois critères suivants sont observés et scorés : l'hémorragie, la coagulation et l'hyperhémie.

Résultats et conclusions :

Le Mélange 1 à 3.75 mg/L ou 5.00 mg/L de substance active peut être classifié comme modérément irritant (Index d'irritation ou IS = $6.8 \pm 0.4$ et $6.3 \pm 0/21$, respectivement), c'est-à-dire qu'il induit une légère lyse (score de sévérité = 1/3) immédiatement après son introduction ou à 30 sec. post-contact, pas d'hémorragie ni coagulation.

Considérant la Q-value (rapport des index entre celui des substances positives de référence NaOH 0.1M [IS = $15 \pm 3$] et SDS 1% [$10 \pm 2$] *vs.* Mélange 1), Q *vs.* NaOH = 0.51 à 0.54 et Q *vs.* SDS = 1.10 à 1.18 pour le Mélange 1 à 3.75 et 5.00 mg/L de substance active, respectivement.

En conséquence, en regard de ce test d'une extrême sensibilité, laquelle est beaucoup plus importante que le test déjà très performant d'irritation vaginal chez la lapine, le Mélange 1 est considéré comme une substance légèrement (3.75 mg/L) à modérément (5.00 mg/L) irritant.

## V - **Étude de la toxicité aigüe par application cutanée et injection intraveineuse - Doses Maximales Tolérées (MTD) et Dose sans effet toxique observable (NOAEL) (Rat; OCDE 474, ICH M3(R2) et S6(R1))**

L'objectif de cette étude est de déterminer la Dose Maximale Tolérée (DMT) du Mélange 1 par application cutanée (1 ml) ou injection intra-caudale (5 ml/kg). Les observations cliniques sont rapportées jusqu'au jour 14.

Conclusions du protocole d'application cutanée :

Le Mélange 1 à une dose de 50 µg/kg de substance active (1 ml à 10 mg/L) est bien toléré. Un léger érythème de la zone traitée est observé ainsi que l'apparition de petites croutes qui disparaissent en 2 jours. Aucune irritation n'est observée.

Les mêmes symptômes proportionnellement plus légers sont observés pour une application du Mélange 1 de 37.5 μg/kg de substance active (1 ml à 7.5 mg/L).

Le Mélange 1 aux doses de 25.0 et 31.3 μg/kg de substance active (1 ml à 5.0 et 6.26 mg/L) est bien toléré. Aucun symptôme cutané, métabolique ou physiologique n'est observé à D14.

La MTD du Mélange 1 en application cutanée chez le rat est de 31.3 μg/kg de substance active (1 ml à 6.26 mg/L).

La NOAEL (Dose sans effect toxique observable) du Mélange 1 en application cutanée chez le rat est de 31.3 μg/kg de substance active (1 ml à 6.26 mg/L).

Conclusions du protocole d'injection intra-caudale :

La difficulté d'une administration intraveineuse du Mélange 1 réside non pas à une toxicité intrinsèque de l'invention mais à sa décomposition au contact du sang qui génère notamment de l'oxygène moléculaire pouvant être responsable d'embolie. La dose limite éthique (3 rats) ayant démontrée un arrêt respiratoire réversible est de 14.6 μg/kg de substance active (5 ml/kg du Mélange 1 à 2.92 mg/L). Aucun signe de toxicité n'a été enregistré jusqu'à D14.

Le Mélange 1 est toléré après une injection intraveineuse à 12.5 μg/kg de substance active (5 ml/kg du Mélange 1 à 2.5 mg/L). Des difficultés physiologiques (mouvements et respiratoires) ont été observées chez l'animal au moment de l'injection. Aucun signe de toxicité n'a été enregistré jusqu'à D14.

Les mêmes symptômes proportionnellement moins importants ont été observés après une injection intraveineuse à 10.4 μg/kg de substance active (5 ml/kg du Mélange 1 à 2.1 mg/L). Aucun signe clinique ni de modification de la courbe du poids n'a été rapporté. Aucun signe de toxicité n'a été enregistré jusqu'à D14. L'observation macroscopique des organes à D14 après autopsie n'a rien révélée.

La MTD du Mélange 1 en injection intra-caudale est de 12.5 μg/kg de substance active (5 ml/kg du Mélange 1 à 2.5 mg/L).

## VI - Étude de la génotoxicité *in vivo* par la recherche de génération de micronuclei après injection intraveineuse du Mélange 1 (Mâles et femelles de rats; OCDE 474 et ICH S2(R1))

Les femelles (n=5) et mâles (n=5) de rat ont été injectés (deux traitements à 22-26 heures d'intervalle; 5 ml/kg) par le véhicule eau HPLC (voie intraveineuse), les témoins positifs: cyclophosphamide à 5 et 10 mg/kg, voie intraveineuse et l'éthyl méthanesulfonate à 100 et 150 mg/kg (voie intrapéritonéale) et le Mélange 1 à 1.46, 4.17 et 12.5 μg/kg de substance active (voie intraveineuse).

La morbidité, la mortalité, le poids des animaux et la clinique (température, peau, poils, yeux, muqueuses, sécrétions et excrétions, fonction respiratoire et neurologique, démarche et posture) ont été notés. 36h à 48h après le second traitement, le sang des animaux a été collecté et analysé en cytométrie de flux (témoin de toxicité : la réduction de la proportion de cellules érythrocytaires immatures CD-71-positives). La proportion d'érythrocytes immature (réticulocytes) micronucléées a été évaluée sur 4000 observations par échantillon sanguin. Chaque population de rats (n=10) pour chaque substance testée représente 40.000 observations (Tab. 5).

Tableau 5 :

| Substance | mg/kg | saxe | n | Réticulocytes (%) | Réticulocytes micronucléés (%) |
|---|---|---|---|---|---|
| Avant traitement | # | mâle | 5 | 4.39±0.92 | 0.15±0.09 |
| | | femelle | 5 | 2.60±0.57 | 0.17±0.07 |
| Contrôle négatif Eau | # | mâle | 5 | 5.17±1.47 | 0.32±0.31 |
| | | femelle | 5 | 3.82±0.29 | 0.12±0.04 |
| Contrôle positif 1 Cyclophosphamide | 5 | mâle | 5 | 3.99±0.49 | 0.56±0.17 |
| | | femelle | 5 | 2.41±0.54 | 0.40±0.13 |
| | 10 | mâle | 5 | 1.95±0.59 | 1.62±0.29 |
| | | femelle | 5 | 1.30±0.43 | 1.02±0.26** |

(suite)

| Substance | mg/kg | saxe | n | Réticulocytes (%) | Réticulocytes micronucléés (%) |
|---|---|---|---|---|---|
| Contrôle positif 2 Méthanesulfonate | 100 | mâle | 5 | 1.41±0.32 | 0.68±0.35** |
| | | femelle | 5 | 1.86±0.25 | 0.48±0.16** |
| | 150 | mâle | 5 | 1.33±0.34 | 0.81±0.48** |
| | | femelle | 5 | 0.45±0.33 | 0.59±0.26** |
| Mélange 1 | $1.46 \cdot 10^{-3\#}$ | mâle | 5 | 5.32±0.76 | 0.10±0.02 |
| | | femelle | 5 | 3.35±0.46 | 0.09±0.03 |
| | $4.17 \cdot 10^{-3\#}$ | mâle | 5 | 4.82±0.41 | 0.11±0.03 |
| | | femelle | 5 | 3.35±0.83 | 0.09±0.02 |
| | $12.5 \cdot 10^{-3\#}$ | mâle | 5 - 3* | 4.79±0.16 | 0.22±0.12 |
| | | femelle | 5 - 1* | 3.40±0.19 | 0.09±0.02 |

* animaux décédés durant le premier traitement par embolie due à la libération d'oxygène moléculaire à partir du Mélange 1 au contact du sang.

** statistiquement différent du groupe Contrôle.

# en substance active $MoO_4^{2-}$

Conclusion : Par rapport aux groupes contrôles (positifs et négatif), le Mélange 1 n'a pas de génotoxicité par induction de production de réticulocytes micronucléés après injection intraveineuse chez les rats (n=10, dont 5 mâles et 5 femelles) d'une gamme de 1.46, 4.17 et 12.5 $\mu$g/kg de substance active.

**Exemple 12** - Comparaison avec d'autres compositions

Le présent exemple montre notamment l'influence de la composition, en particulier du potentiel rédox sur :

1 - le rôle du calcium dans la catalyse Fenton/-Haber-Weiss "like" des formulations testées.
2 - l'existence d'un différentiel entre trois formulations 5.0 mg/L en substance active.

S1 : Mélange 1,
S2 : composition selon US 6,660,289,
S3 : composition selon WO/2010/004161.

**Protocole 1 (Tab. 6 et 7)** :

- 2 paramètres mesurés : i) pH, ii) potentiel rédox.
- catalyseur $CaCl_2$ (1M, en $H_2O$) : ajout en concentration finale de 24mM
- cinétique : initial (T0), 1, 2, 3 minutes (T1, T2, T3) après ajout de $CaCl_2$ sous agitation.

**Résultats et discussion :**

Tableau 6

| Solution S1 | T0 | CaCl₂ 24mM | T1 | T2 | T3 | Moyenne (entre T1 et T3) | Δ / T0 |
|---|---|---|---|---|---|---|---|
| pH | 4.70 | ↓ à T0 | 4.58 | 4.59 | 4.59 | 4.59 sur 3 min. | -0.11 |
| Pot. rédox E° (mV) | 359 | | 383 | 383 | 383 | 383 sur 3 min. | +24 |

| Solution S2 | T0 | CaCl₂ 24mM | T1 | T2 | T3 | Moyenne (entre T1 et T3) | Δ / T0 |
|---|---|---|---|---|---|---|---|
| pH | 2.85 | ↓ à T0 | 2.65 | 265 | 2.66 | 2.65 sur 3 min | -0.20 |
| Pot. rédox E° (mV) | 437 | | 479 | 475 | 474 | 476 sur 3 min | +39 |

| Solution S3 | T0 | CaCl₂ 24mM | T1 | T2 | T3 | Moyenne (entre T1 et T3) | Δ / T0 |
|---|---|---|---|---|---|---|---|
| pH | 3.01 | ↓ à T0 | 2.86 | 2.87 | 2.87 | 2.87 sur 3 min. | -0.14 |
| Pot. rédox E° (mV) | 414 | | 465 | 464 | 464 | 464 sur 3 min | +50 |

Le pH de la peau est de 4.93+/-0.45 à 5.12+/-0.56. Le pH vaginal est normalement de l'ordre de 4.50 et peut aller jusqu'à 6.00 à la ménopause.

- la solution S1 est tamponnée et à un pH de l'ordre de celui de la peau et des muqueuses humaines. Ce pH reste relativement constant jusqu'à 3 min. de contact après ajout de CaCl₂ 24mM final. Il est observé une augmentation modérée de E° pouvant provenir pour part de l'ajout du chlore de CaCl₂.
- Un pH acide amplifie la peroxydation lipidique cytotoxique médiatée par le Fer.

La solution S2 n'est pas tamponnée. Son pH est peu compatible pour un contact avec la peau ou les muqueuses (dont la destruction probable de la flore commensale en utilisation thérapeutique). Ce pH reste relativement constant jusqu'à 3 min. de contact après ajout du CaCl₂ 24mM final. Il est observé une augmentation un peu moins modérée vs. S1 de E° pouvant provenir pour part de l'ajout du chlore de CaCl₂.

La solution S3 n'est pas tamponnée. Son pH est peu compatible pour un contact avec la peau ou les muqueuses (dont la destruction probable de la flore commensale en utilisation thérapeutique). Ce pH reste relativement constant jusqu'à 3 min. de contact après ajout du CaCl₂ 24mM final. Il est observé une augmentation importante (+50mV) de E° pouvant provenir pour part de l'ajout du chlore de CaCl₂ mais en regard de la différence d'augmentation de potentiel *vs.* S1 et S2, il n'y aurait probablement pas que ce seul facteur.

Concernant les ions Fer *in situ,* à pH acide (< 2.33), le potentiel rédox est de l'ordre de 400mV et le Fer sous la forme (III), à savoir sous sa forme la plus cytotoxique. A pH de l'ordre de 4.5, le potentiel rédox est de l'ordre de 300mV, le Fer hémoglobinique sous la forme (II) et le Fer plasmatique complexé à la transferrine sous la forme (III).

Au pH des solutions S2 et S3, la forme oxyde de fer $Fe(OH)_3$ est très défavorisée.. En conséquence, les réactions Fenton-Haber-Weiss sont peu efficaces.

La valeur du potentiel du couple $Fe(OH)_3/Fe^{2+}$ (type Fenton) est : $E'° = 1.19 - 0.18 \times pH$.

Tableau 7

| | subst. act. (mg/L) | pH | $E°_{Fe(OH)3/Fe2+}$ (MV)* | $E°_{solution}$ (mV) |
|---|---|---|---|---|
| **S1** | 5.0 | 4.70 | 344 | 354 |
| **S2** | 5.0 | 2.80 | 686 | 428 |
| S3 | 5.0 | 3.00 | 650 | 418 |
| * niveau cellulaire avec le fer *in situ* | | | | |

D'après cette étude, il y a confirmation que le couple $Fe_{(OH)3}/Fe^{2+}$ est défavorisé. Même si le potentiel rédox de ce couple est amélioré (418-428mV), il reste trop important pour un contact cellulaire.

- le potentiel transmembranaire d'une cellule animale est négatif (≈-70mV). Plus le potentiel d'oxydoréduction de la solution sera important, plus il y aura altération du potentiel transmembranaire et plus il y aura induction du stress oxydatif cellulaire (induction génique de l'apoptose, des voies eNOS, ...) avec concomitamment induction d'un influx calcique lequel potentialise le stress cellulaire important déjà induit par les solutions 2 et 3.

En conséquences, le pH et le potentiel rédox de formulation S2 et surtout le potentiel rédox de la formulation S3 sont très vraisemblablement cytotoxiques. Ces deux solutions S2 et S3 ne peuvent convenir à un usage thérapeutique.

**Protocole 2 (Tab. 8):**

Les concentrations cellulaires et plasmatiques du calcium sont de l'ordre de 2 à 3mM. Ce protocole s'appuie sur une utilisation thérapeutique des formulations.

- 1 paramètre mesuré : quantité résiduelle de peroxydes ($H_2O_2$, acide peracétique; gamme de test Quantofix entre 0 et 25 mg/L).
- catalyseur $CaCl_2$ (1M, en $H_2O$) : ajout en concentration finale de 1.25, 2.5, 10, 25 et 50mM.
- volume final : 1ml = 0.950 $\mu$L de solution testée + 0.05 $\mu$L de la dilution $CaCl_2$. Facteur de dilution de la solution testée : 1.05. Concentration finale de la solution testée 11.4 g/L (12 g/L initial) en traceur $H_2O_2$ contenant 4.76 mg/L de substance active (5.00 mg/L initial).
- cinétique : 5 minutes après ajout de $CaCl_2$ sous agitation.

**Résultats** :

Tableau 8

| Test | Solution testée | [CaCl$_2$] finale (mM) | [peroxydes]$_{finale}$ (mg/L) |
|---|---|---|---|
| 1 | S1 | 50 | >>25 |
| 2 | S1 | 25 | 10-25 |
| 3 | S1 | 10 | 10 |
| 4 | S1 | 2.5 | 5-10 |
| 5 | S1 | 1.25 | ≈25 |
| 6 | S2 | 2.5 | >25 |
| 7 | S3 | 2.5 | >25 |
| 8 | S3 | 1.25 | ≈25 |

La désactivation de la formulation S1 à 5 minutes de contact avec $CaCl_2$ 2.5mM (concentration physiologique) correspond à une décroissance peroxydique d'un facteur 1500 (Test 4), à savoir de 12g/L à 8mg/L en traceur et 5.00 mg/L à 3.33 $\mu$g/L en substance active).

**Conclusions comparatives concernant les formulations S2 et S3**

La solution S2, quel que soit le métal utilisé, n'est pas compatible avec une application thérapeutique, par:

- son pH non tamponnable pour conserver ses potentialités,
- son potentiel rédox cytotoxique,
- sa relative indépendance à la catalyse calcique,
- sa cytotoxicité aux conditions physiologique due à l'$H_2O_2$ et l'acide peracétique (outre la présence d'ions $Ag^+$, par exemple, présentant une cytotoxicité irréversible),
- dans les conditions expérimentales utilisées il n'y a pas de catalyse Fenton-Haber-Weiss like donc il n'y a pas de production radicalaire réactive à utilisation thérapeutique.

La solution S3, quel que soit le métal utilisé et le couple chélatants décrit dans la demande de brevet WO2010/004161, n'est pas compatible avec une application thérapeutique, par:

- son pH non compatible avec le BAPTA,
- son potentiel rédox cytotoxique,
- sa relative indépendance à la catalyse calcique,
- sa cytotoxicité aux conditions physiologique due à l'$H_2O_2$ et l'acide peracétique,
- dans les conditions expérimentales utilisées, il n'y a pas de catalyse Fenton-Haber-Weiss like, donc il n'y a pas de production radicalaire réactive à utilisation thérapeutique.

Au contraire, le modèle chimique réactionnel de la formulation S1 conforme à l'invention, est correct du fait de sa déstabilisation par le calcium.

Pour la formulation S1, les choix :

- du pH,
- du couple tampon (ajout d'un seul élément du couple: l'acide acétique, puisque l'acide peracétique est faiblement produit *in situ*),
- du métal de transition et ses valences compatibles avec les réactions de Fenton-Haber-Weiss like ou non,
- du couple des chélatants (compatibilité entre eux avec des $Kd_{Ca2+}$ et $Kd_{Mo}$, $Kd_{Fe2+/Fe3+}$ en "cascade"),
- la non pénétration cellulaire des chélatants,
- de l'activation du complexe de la solution S1 par le calcium ou d'autres ions métalliques existants *in situ,* à des doses et pH physiologiques (importance du choix des chélatants), à un potentiel rédox limitant les oxydations lipidiques notamment (peu de production de Fe(III)) et la génération instantanée de radicaux réactifs perméants à courte distance, sans accumulation, rendent cette formulation compatible avec une utilisation thérapeutique.

La solution S1 est très fortement dégradée aux concentrations physiologiques de calcium avec un minimum à 2.5mM. Une diminution (1.25mM) ou une augmentation (de 10 à 50mM) de cette concentration calcique non physiologique altère fortement cette dégradation des peroxydes due aux réactions de Fenton-Haber-Weiss like par déplacement des équilibres (loi d'action de masse). Le complexe chimique correspondant à la solution S1 est totalement adapté à une réaction spécifique dans la constante calcique cellulaire et plasmatique.

**Exemple 13** - Exemple de mélange actif selon l'invention avec une combinaison de Mo et La

Le présent exemple de composition (« Mélange 2 ») selon la présente invention est réalisé avec un sel de molybdène et un sel de lanthane. On prépare une composition de formule selon le tableau 9, exprimée en concentration initiale des constituants :

Tableau 9

| **Composants** | **Mélange selon l'invention** |
|---|---|
| Molybdate de sodium | 20.7$\mu$M |
| Nitrate de lanthane | 11.5$\mu$M |
| BAPTA | 42$\mu$M |
| EGTA | 1051.6$\mu$M |
| $H_2O_2$ | 353mM |
| $CH_3COOH/CH_3COONa$ | 70mM |
| pH (par NaOH) | 4.4 - 5.0 |
| $E°_{rédox}$ | 300 à 420mV |

La composition est dite "initiale" car correspondant aux concentrations des réactifs ajoutés sans tenir compte du procédé catalytique mis en oeuvre.
Tous les constituants servant à la synthèse ainsi que le produit fini sont validés par spectre IR-FT.

Tableau 10

| Produit | Pureté | Formule brute | N° CAS |
|---|---|---|---|
| Molybdate de sodium dihydrate | 98-103% | Na2MoO4.2H2O | 10102-40-6 |
| Nitrate de lanthane hexahydrate | ≥99% | La(NO$_3$)$_3$.6H$_2$O | 10277-43-7 |
| BAPTA : (1.2-bis(o-aminophenoxy)ethane-N,N,N',N'-tetraacetic acid) | ≥98% | C22H24N2O10 | 85233-19-8 |

(suite)

| Produit | Pureté | Formule brute | N° CAS |
|---|---|---|---|
| EGTA: (ethylene glycol-bis(2-aminoethylether)-N,N,N',N'-tetraacetic acid) | ≥99% | C14H24N2O10 | 67-42-5 |
| Peroxyde d'hydrogène | 29-31% | H2O2 | 722-84-1 |
| Acide acétique glacial* | 99.8-100.5% | CH3COOH | 64-19-7 |
| Acétate de sodium trihydrate | 99-101% | C2H3NaO2.3H2O | 5010524 |
| Sodium hydroxyde | 10N | NaOH | 1310-73-2 |
| Eau déminéralisée | 1µS | H2O | 7732-18-5 |

La substance active est : $La(MoO_4)^{1-}$, $Na^+$ (hydroperoxomolybdate de lanthane).

Poids Moléculaire : 481.81 g/mole.

**Procédé de fabrication** :

Le procédé de fabrication est identique à celui du Mélange 1 selon l'exemple 1, à l'exception des modifications décrites ci-dessous :
• Deux solutions SC sont préparées :

Solution A - $Na_2MoO_4.2H_2O$ (source de Mo(VI) ; 100%)     200mg (2.4mM final)
Eau                                                          qsp 340ml

Agitation douce, température ambiante

Solution B - $La(NO_3)_3.6H_2O$ (source de La(III) ; 100%)     200mg (1.4mM final)
Eau                                                            qsp 340ml

Agitation douce, température ambiante
• La solution A (10 mL) puis la solution B (10 mL) sont introduites progressivement dans la solution Si2 pour préparer une solution S1 d'hydro-peroxomolybdate de lanthane
• ETAPE (vii) : Ajout des agents chélatants - Préparation d'une solution S4

Introduction BAPTA (98.8%)     20mg/L,
Introduction EGTA (99.1%)     400mg/L

• Le pH de la solution finale est ajusté par NaOH à 4,67
• Le $E°_{rédox}$ final est : 380 mV
Dans le Mélange 2, la concentration finale en $La(MoO_4)^{1-}$, $Na^+$ est : 20.8µM.

**Exemple 14** -- Evaluation de l'efficacité oxydoréductrice de l'invention en double complexe molybdate et lanthanate sur plasma humain

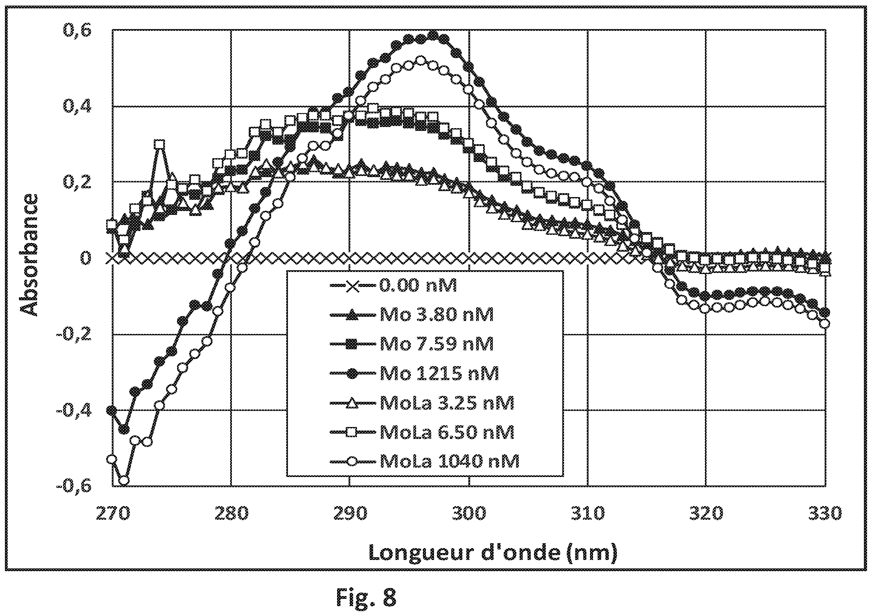

De façon à comparer l'efficacité oxydative de la formulation à base de $(MoO_4)^{2-}$ (Mélange 1) et celle à base de $La(MoO_4)^{1-}$ (Mélange 2), des formulations de concentrations comparables à celles décrites à la Fig. 3 et inférieures à celles décrites à la Fig. 5 ont été mise en contact 2 min. avec du plasma humain. Après soustraction des blancs expérimentaux, spécifiquement dans cette zone de concentrations, les Mélanges 1 et 2 selon l'invention génèrent une augmentation de la production *in situ* de quercétine oxydée. Les concentrations finales de $MoO_4^{2-}$ et de $La(MoO_4)^{1-}$ testées étaient alors de : 0.00, 3.80, 7.59, 1215 nM et 0.00, 3.25, 6.50, 1040 nM, respectivement (Fig. 8).

La différence de production de quercétine oxydée (apex vers 295 nm) entre les deux formulations (Mélange 1 et Mélange 2) n'est pas significative. L'efficacité oxydoréductrice entre les deux compositions selon l'invention est comparable.

**Revendications**

1. Composition ou mélange, de préférence thérapeutiquement actif par voie topique, comprenant :

   - au moins un sel de métal, le métal étant choisi parmi le molybdène (Mo), le tungstène (W), le vanadium (V), l'or (Au), un lanthanide, en particulier le lanthane;
   - au moins un agent chélatant choisi parmi BAPTA, EGTA et l'un quelconque de leurs mélanges;
   - au moins une source de radicaux peroxydant;
   - au moins un agent tampon comprenant l'acide acétique,
   ledit mélange ou ladite composition présentant un potentiel rédox de 250 à 550 millivolts, mesuré avec un pH mètre / rédox mètre combiné, équipé d'une électrode Platine / calomel étalonnée.

2. Composition ou Mélange, selon la revendication 1, **caractérisé en ce que** ledit mélange ou ladite composition présente un potentiel rédox de 300 à 450 millivolts et encore de préférence de 300 à 420 millivolts.

3. Composition ou Mélange, selon la revendication 1 ou 2, **caractérisé en ce que** le sel de métal comprend un peroxométallate ou hydro-peroxométallate et le métal du métallate est de valence maximale.

4. Composition ou mélange, selon l'une quelconque des revendications 1 à 3, **caractérisé en ce que** le métal est oxydé à son degré d'oxydation maximal.

5. Composition ou mélange, selon l'une quelconque des revendications 1 à 4, **caractérisé en ce que** le sel métallique est un sel de molybdène ou comprenant du molybdène ou un sel de lanthanide ou comprenant du lanthanide, ou est un mélange de sel de molybdène et de sel de lanthanide.

6. Composition ou mélange, selon l'une quelconque des revendications 1 à 5, **caractérisé en ce que** le sel de molybdène est de préférence d'unité peroxo ou hydroperoxo.

7. Composition ou mélange, selon l'une quelconque des revendications 1 à 6, **caractérisé en ce que** la substance active est de type $MoO_4^{2-}$ ou sa forme hydroperoxo.

8. Composition ou mélange, selon l'une quelconque des revendications 1 à 7, **caractérisé en ce qu'**il comprend au moins deux agents chélatants.

9. Composition ou mélange, selon l'une quelconque des revendications 1 à 10, **caractérisé en ce que** le ou les agents chélatants présentent au moins trois groupements de coordination, de préférence des groupements acides carboxyliques, à trois extrémités de la ou des molécules formant le ou les agents chélatants, lesdits groupements de coordination étant séparés par une chaine d'au moins trois atomes dont au moins un atome d'azote, de préférence l'agent chélatant est choisi parmi BAPTA, EGTA et l'un quelconque de leurs mélanges.

10. Composition ou mélange, selon l'une quelconque des revendications 1 à 9, **caractérisé en ce que** la source principale de radicaux peroxydant est présente à une concentration allant de 200 à 600 mM, de préférence de 300 à 500 mM, et encore de préférence de 340 à 450 mM.

11. Composition ou mélange, selon l'une quelconque des revendications 1 à 10, **caractérisé en ce que** sel de métal comprend un peroxométallate ou hydro-peroxométallate $(Mo_2O_6)^{4+}$ ou $[Mo_4O_{12}(O_2)_2]^{4+}$.

12. Composition ou mélange, selon l'une quelconque des revendications 1 à 10, **caractérisé en ce que** le sel métallique et les agents chélatants sont présents en quantité et ratio suffisant pour la production de complexes {peroxo-métallate - agent(s) chélatant(s)}.

13. Composition ou mélange, selon l'une quelconque des revendications 1 à 10, **caractérisé en ce qu'**il comprend un sel, de préférence de sodium, de molybdène, un mélange de deux agents chélatants du type BAPTA et EGTA, du peroxyde d'hydrogène et un agent tampon acétate.

14. Composition ou mélange, selon l'une quelconque des revendications 1 à 13, **caractérisé en ce que** le mélange comprend comme agent tampon autorisant un pH 4,0 à 5,2,.

**15.** Composition ou mélange, selon l'une quelconque des revendications 1 à 14, **caractérisé en ce qu'**il comprend de 0,1 à 100 $\mu$M, et de préférence de 1 à 50 $\mu$M, et encore de préférence de 5 à 30 $\mu$M de sel métallique de molybdène.

**16.** Composition ou mélange, selon l'une quelconque des revendications 1 à 15, **caractérisé en ce qu'**il comprend de 0,1 à 100 $\mu$M, et de préférence de 1 à 50 $\mu$M, et encore de préférence de 5 à 20 $\mu$M de BAPTA ou de 20 à 80 $\mu$M de BAPTA.

**17.** Composition ou mélange, selon l'une quelconque des revendications 1 à 16, **caractérisé en ce qu'**il comprend de 0,1 à 1 mM, et de préférence de 50 à 800 $\mu$M, et encore de préférence de 200 à 700 $\mu$M d'EGTA ou de 600 à 1400 $\mu$M d'EGTA.

**18.** Composition ou mélange, selon l'une quelconque des revendications 1 à 17, **caractérisé en ce qu'**il comprend un sel de molybdène et les agents chélatants selon un ratio 10/1 à 1/100, dans lequel le ratio : sel de Mo/agent chélatant est exprimé en concentrations molaires.

**19.** Composition ou mélange, selon l'une quelconque des revendications 1 à 18, **caractérisé en ce qu'**il comprend du peroxyde d'hydrogène présent à une concentration allant de 200 à 600 mM, de préférence de 300 à 500 mM, et encore de préférence de 340 à 450 mM.

**20.** Composition ou mélange, selon l'une quelconque des revendications 1 à 19, **caractérisé en ce qu'**il comprend :

- sel de Molybdate, et par exemple Molybdate de sodium;
- BAPTA;
- EGTA;
- $H_2O_2$;
- CH3COOH/CH3COONa;
- $E_{rédox}$ de 250 à 550 mV, et de préférence de 300 à 450 mV, et encore de préférence de 300 à 420mV;
- pH 4.4 - 5.0.

**21.** Composition ou mélange, selon l'une quelconque des revendications 1 à 19, **caractérisé en ce qu'**il comprend :

- sel de Molybdate, et par exemple Molybdate de sodium;
- sel de Lanthane, et par exemple Nitrate de Lanthane;
- BAPTA;
- EGTA;
- $H_2O_2$;
- CH3COOH/CH3COONa;
- $E_{rédox}$ de 250 à 550 mV, et de préférence de 300 à 450 mV, et encore de préférence de 300 à 420mV;
- pH 4.4 - 5.0.

**22.** Procédé de préparation d'un mélange ou d'une composition tel que défini selon l'une quelconque des revendications 1 à 21, **caractérisé en ce qu'**il comprend (i) la préparation d'une solution tampon (ST) comprenant un agent tampon présentant un pH acide, (ii) la préparation d'une solution d'un complexe métallique (SC) comprenant un sel d'oxyde métallique, (iii) la préparation d'une première solution initiale (Si1) comprenant du peroxyde d'hydrogène, (iv) la préparation d'une seconde solution initiale (Si2) par mélange de la solution ST avec la solution Si1, (v) la préparation d'une solution (S1) comprenant un composé peroxo-métallique par mélange de la solution SC avec la solution Si2, (vi) la préparation d'une solution S2 par ajustement du pH de la solution S1 avec une base, le pH de la solution S2 étant plus basique que le pH de la solution ST, (vii) l'ajout à la solution S2 d'un ou plusieurs agents chélatants, (viii) l'ajustement éventuel du pH, (ix) l'ajustement éventuel du volume de la solution finale, l'obtention d'un mélange ou d'une composition tel que défini selon l'une quelconque des revendications 1 à 21.

**23.** Procédé, selon la revendication 15, **caractérisé en ce que** le peroxyde d'hydrogène est présent dans la solution Si1 à une concentration allant de 200 à 600 mM, de préférence de 300 à 500 mM, et encore de préférence de 330 à 460 mM.

**24.** Procédé, selon la revendication 22 ou 23, **caractérisé en ce que** l'agent tampon de la solution ST est un tampon acide carboxylique/carboxylate, et la solution ST présente un ratio peroxyde d'hydrogène/carboxylate compris entre 20/1 et 1/1, et de préférence d'environ 5/1.

**25.** Procédé, selon l'une quelconque des revendications 22 à 24, **caractérisé en ce que** le procédé comprend une mesure du potentiel rédox aux étapes (iii) à (ix), et de préférence comprend également une mesure du pH aux étapes (iii) à (ix).

**26.** Procédé, selon l'une quelconque des revendications 22 à 25, **caractérisée en ce que** le composé peroxo-métallique de la solution S1 est un composé peroxomolybdique.

**27.** Composition pharmaceutique à usage topique, **caractérisée en ce qu'**elle comprend un mélange ou composition thérapeutiquement actif, tel que défini selon l'une quelconque des revendications 1 à 21, ou susceptible d'être obtenu selon un procédé tel que défini à l'une quelconque des revendications 22 à 26.

**28.** Composition pharmaceutique à usage topique selon la revendication 27 , **caractérisée en ce qu'**elle comprend de 0,001 à 5 mM, de préférence de 0,01 à 2 mM, et encore de préférence de 0,02 à 1 mM du mélange pharmaceutiquement actif.

**29.** Composition pharmaceutique à usage topique l'une quelconque des revendications 27 à 28, pour son utilisation dans une méthode de traitement thérapeutique d'une infection virale, et en particulier impliquant un virus de la famille *Herpesviridae.*

**30.** Composition pharmaceutique, selon l'une quelconque des revendications 27 à 29, pour son utilisation dans une méthode de traitement thérapeutique d'une infection impliquant HSV-1 et/ou HSV-2.

**31.** Composition pharmaceutique, selon l'une quelconque des revendications 27 à 30, pour son utilisation dans une méthode de traitement thérapeutique anti-inflammatoire.

**32.** Composition pharmaceutique, selon l'une quelconque des revendications 27 à 31, pour son utilisation dans une méthode de traitement thérapeutique préventif ou curatif.

**Patentansprüche**

**1.** Zusammensetzung oder Gemisch, vorzugsweise therapeutisch wirksam auf topischem Weg, umfassend:

- mindestens ein Metallsalz, wobei das Metall ausgewählt ist aus Molybdän (Mo), Wolfram (W), Vanadium (V), Gold (Au), einem Lanthanid, insbesondere Lanthan;
- mindestens einen Chelatbildner, der ausgewählt ist aus BAPTA, EGTA und einem beliebigen Gemisch davon;
- mindestens eine Quelle für Peroxidradikale;
- mindestens ein Puffermittel, umfassend Essigsäure,
wobei das Gemisch oder die Zusammensetzung ein Redoxpotential von 250 bis 550 Millivolt aufweist, gemessen mit einem kombinierten pH-Meter/Redoxmeter, das mit einer kalibrierten Platin/Kalomel-Elektrode ausgestattet ist.

**2.** Zusammensetzung oder Gemisch nach Anspruch 1, **dadurch gekennzeichnet, dass** das Gemisch oder die Zusammensetzung ein Redoxpotential von 300 bis 450 Millivolt und bevorzugter von 300 bis 420 Millivolt aufweist.

**3.** Zusammensetzung oder Gemisch nach Anspruch 1 oder 2, **dadurch gekennzeichnet, dass** das Metallsalz ein Peroxometallat oder Hydroperoxometallat umfasst und das Metall des Metallats maximalwertig ist.

**4.** Zusammensetzung oder Gemisch nach einem der Ansprüche 1 bis 3, **dadurch gekennzeichnet, dass** das Metall auf seinen maximalen Oxidationsgrad oxidiert wird.

**5.** Zusammensetzung oder Gemisch nach einem der Ansprüche 1 bis 4, **dadurch gekennzeichnet, dass** das Metallsalz ein Molybdänsalz oder Molybdän umfassend oder ein Lanthanidsalz oder Lanthanid umfassend ist, oder ein Gemisch aus Molybdänsalz und Lanthanidsalz ist.

**6.** Zusammensetzung oder Gemisch nach einem der Ansprüche 1 bis 5, **dadurch gekennzeichnet, dass** das Molybdänsalz vorzugsweise eine Peroxo- oder Hydroperoxo-Einheit ist.

7. Zusammensetzung oder Gemisch nach einem der Ansprüche 1 bis 6, **dadurch gekennzeichnet, dass** der Wirkstoff vom Typ $MoO_4^{2-}$ oder seine Hydroperoxoform ist.

8. Zusammensetzung oder Gemisch nach einem der Ansprüche 1 bis 7, **dadurch gekennzeichnet, dass** sie/es mindestens zwei Chelatbildner umfasst.

9. Zusammensetzung oder Gemisch nach einem der Ansprüche 1 bis 10, **dadurch gekennzeichnet, dass** der oder die Chelatbildner mindestens drei Koordinationsgruppierungen, vorzugsweise Carbonsäuregruppierungen, an drei Enden des oder der Moleküle aufweisen, die den oder die Chelatbildner bilden, wobei die Koordinationsgruppierungen durch eine Kette von mindestens drei Atomen, darunter mindestens ein Stickstoffatom, getrennt sind, vorzugsweise wobei der Chelatbildner ausgewählt ist aus BAPTA, EGTA und einem beliebigen Gemisch davon.

10. Zusammensetzung oder Gemisch nach einem der Ansprüche 1 bis 9, **dadurch gekennzeichnet, dass** die Hauptquelle für Peroxidationsradikale in einer Konzentration von 200 bis 600 mM, vorzugsweise von 300 bis 500 mM und bevorzugter von 340 bis 450 mM vorhanden ist.

11. Zusammensetzung oder Gemisch nach einem der Ansprüche 1 bis 10, **dadurch gekennzeichnet, dass** das Metallsalz ein Peroxometallat oder Hydroperoxometallat $(Mo_2O_6)^{4+}$ oder $[Mo_4O_{12}(O_2)_2]^{4+}$ umfasst.

12. Zusammensetzung oder Gemisch nach einem der Ansprüche 1 bis 10, **dadurch gekennzeichnet, dass** das Metallsalz und die Chelatbildner in einer Menge und einem Verhältnis vorhanden sind, die für die Herstellung von Komplexen {Peroxometallat - Chelatbildner(n)} ausreichend sind.

13. Zusammensetzung oder Gemisch nach einem der Ansprüche 1 bis 10, **dadurch gekennzeichnet, dass** sie/es ein Salz, vorzugsweise Natriumsalz, Molybdänsalz, ein Gemisch aus zwei Chelatbildnern vom Typ BAPTA und EGTA, Wasserstoffperoxid und ein Acetat-Puffermittel umfasst.

14. Zusammensetzung oder Gemisch nach einem der Ansprüche 1 bis 13, **dadurch gekennzeichnet, dass** das Gemisch als Puffermittel umfasst, das einen pH von 4,0 bis 5,2 zulässt.

15. Zusammensetzung oder Gemisch nach einem der Ansprüche 1 bis 14, **dadurch gekennzeichnet, dass** sie/es 0,1 bis 100 $\mu$M, und vorzugsweise 1 bis 50 $\mu$M, und bevorzugter 5 bis 30 $\mu$M Molybdänmetallsalz umfasst.

16. Zusammensetzung oder Gemisch nach einem der Ansprüche 1 bis 15, **dadurch gekennzeichnet, dass** sie/es 0,1 bis 100 $\mu$M, und vorzugsweise 1 bis 50 $\mu$M, und bevorzugter 5 bis 20 $\mu$M BAPTA oder 20 bis 80 $\mu$M BAPTA umfasst.

17. Zusammensetzung oder Gemisch nach einem der Ansprüche 1 bis 16, **dadurch gekennzeichnet, dass** sie/es 0,1 bis 1 mM, und vorzugsweise 50 bis 800 $\mu$M, und bevorzugter 200 bis 700 $\mu$M EGTA oder 600 bis 1400 $\mu$M EGTA umfasst.

18. Zusammensetzung oder Gemisch nach einem der Ansprüche 1 bis 17, **dadurch gekennzeichnet, dass** sie/es ein Molybdänsalz und die Chelatbildner in einem Verhältnis von 10/1 bis 1/100 umfasst, wobei das Verhältnis: Mo-Salz/Chelatbildner in molaren Konzentrationen ausgedrückt ist.

19. Zusammensetzung oder Gemisch nach einem der Ansprüche 1 bis 18, **dadurch gekennzeichnet, dass** es Wasserstoffperoxid umfasst, das in einer Konzentration von 200 bis 600 mM, vorzugsweise von 300 bis 500 mM und bevorzugter von 340 bis 450 mM vorhanden ist.

20. Zusammensetzung oder Gemisch nach einem der Ansprüche 1 bis 19, **dadurch gekennzeichnet, dass** sie/es Folgendes umfasst:

   - Molybdat-Salz und beispielsweise Natrium-Molybdat;
   - BAPTA;
   - EGTA;
   - $H_2O_2$;
   - CH3COOH/CH3COONa;
   - $E_{redox}$ von 250 bis 550 mV, vorzugsweise von 300 bis 450 mV und bevorzugter von 300 bis 420mV;
   - pH 4,4-5,0.

**21.** Zusammensetzung oder Gemisch nach einem der Ansprüche 1 bis 19, **dadurch gekennzeichnet, dass** sie/es Folgendes umfasst:

- Molybdat-Salz und beispielsweise Natrium-Molybdat;
- Lanthansalz und beispielsweise Lanthannitrat;
- BAPTA;
- EGTA;
- $H_2O_2$;
- CH3COOH/CH3COONa;
- $E_{redox}$ von 250 bis 550 mV, vorzugsweise von 300 bis 450 mV und bevorzugter von 300 bis 420 mV;
- pH 4,4-5,0.

**22.** Verfahren zur Herstellung eines Gemischs oder einer Zusammensetzung nach einem der Ansprüche 1 bis 21, **dadurch gekennzeichnet, dass** es Folgendes umfasst: (i) Herstellen einer Pufferlösung (ST) umfassend ein Puffermittel, das einen sauren pH aufweist, (ii) Herstellen einer Metallkomplexlösung (SC) umfassend ein Metalloxidsalz, (iii) Herstellen einer ersten Ausgangslösung (Si1) umfassend Wasserstoffperoxid, (iv) Herstellen einer zweiten Ausgangslösung (Si2) durch Mischen der Lösung ST mit der Lösung Si1, (v) Herstellen einer Lösung (S1) umfassend eine Peroxo-Metallverbindung durch Mischen der Lösung SC mit der Lösung Si2, (vi) Herstellen einer Lösung S2 durch Einstellen des pHs der Lösung S1 mit einer Base, wobei der pH der Lösung S2 basischer ist als der pH der Lösung ST, (vii) Hinzufügen eines oder mehrerer Chelatbildner zu der Lösung S2, (viii) gegebenenfalls Einstellen des pHs, (ix) gegebenenfalls Einstellen des Volumens der Endlösung, Erlangen eines Gemischs oder einer Zusammensetzung, wie sie nach einem der Ansprüche 1 bis 21 definiert ist.

**23.** Verfahren nach Anspruch 15, **dadurch gekennzeichnet, dass** das Wasserstoffperoxid in der Si1-Lösung in einer Konzentration von 200 bis 600 mM, vorzugsweise von 300 bis 500 mM und bevorzugter von 330 bis 460 mM vorhanden ist.

**24.** Verfahren nach Anspruch 22 oder 23, **dadurch gekennzeichnet, dass** das Puffermittel der Lösung ST ein Carbonsäure/Carboxylat-Puffer ist und die Lösung ST ein Verhältnis Wasserstoffperoxid/Carboxylat von zwischen 20:1 und 1:1, vorzugsweise etwa 5:1, aufweist.

**25.** Verfahren nach einem der Ansprüche 22 bis 24, **dadurch gekennzeichnet, dass** das Verfahren eine Messung des Reoxpotentials in den Schritten (iii) bis (ix) umfasst, und vorzugsweise auch eine Messung des pHs in den Schritten (iii) bis (ix) umfasst.

**26.** Verfahren nach einem der Ansprüche 22 bis 25, **dadurch gekennzeichnet, dass** die Peroxo-Metallverbindung der Lösung S1 eine Peroxo-Molybdänverbindung ist.

**27.** Pharmazeutische Zusammensetzung zur topischen Verwendung, **dadurch gekennzeichnet, dass** sie ein therapeutisch wirksames Gemisch bzw. Zusammensetzung umfasst, wie sie in einem der Ansprüche 1 bis 21 definiert ist oder nach einem Verfahren, wie es in einem der Ansprüche 22 bis 26 definiert ist, erlangt werden kann.

**28.** Pharmazeutische Zusammensetzung zur topischen Verwendung nach Anspruch 27, **dadurch gekennzeichnet, dass** sie 0,001 bis 5 mM, vorzugsweise 0,01 bis 2 mM und bevorzugter 0,02 bis 1 mM des pharmazeutisch wirksamen Gemischs umfasst.

**29.** Pharmazeutische Zusammensetzung zur topischen Verwendung nach einem der Ansprüche 27 bis 28 zur Verwendung in einem Verfahren zur therapeutischen Behandlung einer Virusinfektion, insbesondere unter Beteiligung eines Virus der Familie *Herpesviridae.*

**30.** Pharmazeutische Zusammensetzung nach einem der Ansprüche 27 bis 29 zur Verwendung in einem Verfahren zur therapeutischen Behandlung einer Infektion, an der HSV-1 und/oder HSV-2 beteiligt sind.

**31.** Pharmazeutische Zusammensetzung nach einem der Ansprüche 27 bis 30 zur Verwendung in einem entzündungshemmenden therapeutischen Behandlungsverfahren.

**32.** Pharmazeutische Zusammensetzung nach einem der Ansprüche 27 bis 31 zur Verwendung in einem vorbeugenden oder heilenden therapeutischen Behandlungsverfahren.

**Claims**

1. A composition or mixture, preferably therapeutically active via topical route, comprising:

   - at least one metal salt, the metal being selected from molybdenum (Mo), tungsten (W), vanadium (V), gold (Au), lanthanide, in particular lanthanum;
   - at least one chelating agent selected from among BAPTA, EGTA and any mixtures thereof;
   - at least one source of peroxidizing radicals;
   - at least one buffering agent comprising acetic acid,

   said composition or mixture having a redox potential from 250 to 550 millivolts, measured with a combined pH-meter / redox-meter, equipped with a calibrated platinum / calomel electrode.

2. The composition or mixture, according to claim 1, **characterized in that** said composition or mixture has a redox potential from 300 to 450 millivolts, and even more preferably from 300 to 420 millivolts.

3. The composition or mixture, according to claim 1 or 2, **characterized in that** the metal salt comprises a peroxo-metallate or hydro-peroxometallate and the metallate is of maximum valency.

4. The composition or mixture, according to any of claims 1 to 3, **characterized in that** the metal is oxidized to its maximum degree of oxidation.

5. The composition or Mixture, according to any of claims 1 to 4, **characterized in that** the metal salt is a salt of molybdenum or comprising molybdenum or a lanthanide salt or comprising lanthanide, or is a mixture of a molybdenum salt and lanthanide salt.

6. The composition or Mixture, according to any of claims 1 to 5, **characterized in that** the molybdenum salt is preferably a peroxo or hydroperoxo unit.

7. The composition or mixture, according to any of claims 1 to 6, **characterized in that** the active substance is of the $MoO_4^{2-}$ type or its hydro-peroxo form.

8. The composition or mixture, according to any of claims 1 to 7, **characterized in that** it comprises at least two chelating agents.

9. The composition or mixture, according to any of claims 1 to 10, **characterized in that** the chelating agent(s) has (have) at least three coordination groups, preferably carboxylic acid groups, at three ends of the molecule(s) forming the chelating agent(s), said coordination groups being separated by a chain of at least three atoms including at least at nitrogen atom, preferably the chelating agent is selected from among BAPTA, EGTA and any mixtures thereof.

10. The composition or mixture, according to any of claims 1 to 9, **characterized in that** the main source of peroxidizing radicals is present at a concentration ranging from 200 to 600 mM, preferably from 300 to 500 mM, and still preferably from 340 to 450 mM.

11. The composition or mixture, according to any of claims 1 to 10, **characterized in that** the metal salt comprises a peroxometallate or hydroxometallate $(Mo_2O_6)^{4+}$ or $[Mo_4O_{12}(O_2)_2]^{4+}$.

12. The composition or mixture, according to any of claims 1 to 10, **characterized in that** the metal salt and the chelating agents are present in a sufficient amount and ratio for producing {peroxo-metallate - chelating} agent(s)} complexes.

13. The composition or mixture, according to any of claims 1 to 10, **characterized in that** it comprises a salt, preferably a sodium salt, of molybdenum, a mixture of two chelating agents of BAPTA and EGTA types, hydrogen peroxide and an acetate buffer.

14. The composition or mixture, according to any of claims 1 to 13, **characterized in that** the mixture comprises a buffering agent allowing a pH of 4.0 to 5.2.

15. The composition or mixture, according to any of claims 1 to 14, **characterized in that** it comprises from 0.1 to 100 $\mu$M, and preferably from 1 to 50 $\mu$M, and more preferably from 5 to 30 $\mu$M of metal salt of molybdenum.

16. The composition or mixture, according to any of claims 1 to 15, **characterized in that** it comprises from 0.1 to 100 μM, and preferably from 1 to 50 μM, and more preferably from 5 to 20 μM of BAPTA or from 20 to 80 μM of BAPTA.

17. The composition or mixture, according to any of claims 1 to 16, **characterized in that** it comprises from 0.1 to 1 mM, and preferably from 50 to 800 μM, and more preferably from 200 to 700 μM of EGTA or 600 to 1400 μM of EGTA.

18. The composition or mixture, according to any of claims 1 to 17, **characterized in that** it comprises a molybdenum salt and chelating agents according to a ratio from 10/1 to 1/100, in which said ration Mo salt/chelating agent is expressed in molar concentrations.

19. The composition or mixture, according to any of claims 1 to 18, **characterized in that** it comprises hydrogene peroxide present at a concentration from 200 to 600 mM, preferably from 300 to 500mM, and more preferably from 340 to 450 mM.

20. The composition or mixture, according to any of claims 1 to 19, **characterized in that** it comprises :

   - molybdenum salt, and for example sodium molybdate ;
   - BAPTA;
   - EGTA;
   - $H_2O_2$ ;
   - CH3COOH/CH3COONa ;
   - $E_{redox}$ from 250 to 550 mV, preferably from 300 to 450 mV, and more preferably from 300 to 420 mV ;
   - pH 4.4-5.0

21. The composition or mixture, according to any of claims 1 to 19, **characterized in that** it comprises :

   - molybdenum salt, and for example sodium molybdate ;
   - lanthanum salt, and for example lanthanum nitrate ;
   - BAPTA;
   - EGTA;
   - $H_2O_2$ ;
   - CH3COOH/CH3COONa ;
   - $E_{redox}$ from 250 to 550 mV, preferably from 300 to 450 mV, and more preferably from 300 to 420 mV ;
   - pH 4.4-5.0.

22. A method for preparing a mixture or a composition as defined according to any of claims 1 to 21, **characterized in that** it comprises (i) the preparation of a buffering solution (BS) comprising a buffering agent having an acid pH, (ii) the preparation of a solution of a metal complex (SC) comprising a metal oxide salt, (iii) the preparation of a first initial solution (Si1) comprising hydrogen peroxide, (iv) the preparation of a second initial solution (Si2) by mixing the BS solution with the Si1 solution, (v) the preparation of a solution (S1) comprising a peroxo-metal compound by mixing the CS solution with the Si2 solution, (vi) the preparation of a solution S2 by adjusting the pH of the solution S1 with a base, the pH of the solution S2 being more basic than the pH of the solution BS, (vii) the addition to the solution S2 of one or several chelating agents, (viii) the optional adjustment of the pH, (ix) the optional adjustment of the volume of the final solution, the obtaining of a mixture or of a composition as defined according to any of claims 1 to 21.

23. The method, according to claim 22, **characterized in that** the hydrogen peroxide is present in the Si1 solution at a concentration ranging from 200 to 600 mM, preferably from 300 to 500 mM, and still preferably from 330 to 460 mM.

24. The method, according to claim 22 or 23, **characterized in that** the buffering agent of the BS solution is a carboxylic acid/carboxylate buffer, and the BS solution has a hydrogen peroxide/carboxylate ratio comprised between 20/1 and 1/1, and preferably of about 5/1.

25. The method, according to any of claims 22 to 24, **characterized in that** the method comprises a measurement of the redox potential in steps (iii) to (ix), and preferably also comprises a measurement of the pH in steps (iii) to (ix).

26. The method, according to any of claims 22 to 25, **characterized in that** the peroxo-metal compound of the solution S1 is a peroxo-molybdenum compound.

27. A pharmaceutical composition for topical use, **characterized in that** it comprises a therapeutically active mixture or composition, as defined according to any of claims 1 to 21, or which may be obtained according to a method as defined in any of claims 22 to 26.

28. The pharmaceutical composition for topical use according to claim 27, **characterized in that** it comprises from 0.001 to 5 mM, preferably from 0.01 to 2 mM, and still preferably from 0.02 to 1 mM of the pharmaceutically active mixture.

29. The pharmaceutical composition for topical use according to any one of claims 27 to 28, for its use in a method for therapeutic treatment of a viral infection, and in particular involving a virus from the *Herpesviridae* family.

30. The pharmaceutical composition according to any of claims 27 to 29, for its use in a method for therapeutic treatment of an infection involving HSV-1 and/or HSV-2.

31. The pharmaceutical composition, according to any of claims 27 to 30, for its use in a method for anti-inflammatory therapeutic treatment.

32. The pharmaceutical composition, according to any of claims 27 to 31 for its use in a method for preventive or curative therapeutic treatment.

Fig. 1

Fig. 2

Fig. 3

EP 3 435 979 B1

**AUC$_{285-305nm}$ = f([Mélange 1])**

$$y = 0{,}0144x^2 + 0{,}5863x + 0{,}4511$$
$$R^2 = 0{,}9934$$

AUC$_{285-305nm}$

[Mélange]$_{final}$ (MoO$_4^{2-}$; nM)

EP 3 435 979 B1

Fig. 4

Fig. 5

Fig. 6

**Fig. 7**

Fig. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010004161 A2
- US 2007059255 A1
- WO 2005010774 A1

- US 6660289 B
- WO 2010004161 A

**Littérature non-brevet citée dans la description**

- Le virus de l'herpès. Organisation Mondiale de la Santé (OMS), Janvier 2016
- MMW R Morb Mortal Wkly Rep. Centers for Disease Control and Prévention (CDC), 2010, vol. 59, 456-459
- **BACON T.H. et al.** *Clin. Microbiol. Rev.,* 2003, vol. 16 (1), 114-128
- **BISWAS S. et al.** *Antiviral Res.,* 2008, vol. 80 (1), 81-85
- **VOIR OYERINDE OYEYEMI F. et al.** Solution structure of molybdic acid from Raman spectroscopy and DFT analysis. *Inorganica Chimica Acta,* 2008, vol. 361, 1000-1007
- **VOIR SUPONITSKIY, Y.L. ; PROSHINA, O.P. ; DYUNIN, A.G. et al.** Thermodynamic properties of lanthanum Molybdates. *Russ. J. Phys. Chem.,* 2016, vol. 90, 267
- **EL HAJJI H. et al.** Interactions of quercetin with iron and copper ions: Complexation and autoxidation. *Free Radic. Res.,* 2006, vol. 40 (3), 303-320
- **BALCERZAK M. et al.** Sélective determination of Fe(III) in Fe(II) samples by UV-spectrophotometry with the aid of quercetin and morin. *Acta Pharm.,* 2008, vol. 58, 327-334
- **DEMENT'EV I.A. et al.** Mononuclear, polynuclear, and cluster complexes of molybdenum and their reactions as models of biochemical systems and processes. *Russ. J. Gen. Chem.,* 2007, vol. 77 (5), 822-843

- **NOBLE R.W. et al.** The reaction of ferrous horseradish peroxidase with hydrogen peroxide. *J. Biol. Chem.,* 1970, vol. 245 (9), 2409-2413
- **KESSLER H.H. et al.** Détection of Herpes Simplex Virus DNA by real-time PCR. *J Clin Microbiol.,* 2000, vol. 38 (7), 2638-2642
- **MULLIS K. et al.** Spécifie Enzymatic Amplification of DNA In Vitro: The Polymerase Chain Reaction. *Cold Spring Harb. Symp. Quant. Biol.,* 1986, vol. 51, 263-273
- **KESSLER H.H. et al.** Détection of Herpes simplex virus DNA by real-time PCR. *J. Clin. Microbiol.,* 2000, vol. 38 (7), 2638-2642
- **MIKLOSKA Z. et al.** Alpha and Gamma Interferons Inhibit Herpes Simplex Virus Type 1 Infection and Spread in Epidermal Cells after Axonal Transmission. *J. Virol.,* 2001, vol. 75 (23), 11821-11826
- **SAINZ JR. B. et al.** AlphalBeta Interferon and Gamma Interferon Synergize To Inhibit the Réplication of Herpes Simplex Virus Type 1. *J. Virol.,* 2002, vol. 76 (22), 11541-11550
- **MAGNUSSON B et al.** The identification of contact allergens by animal assay. The guinea pig maximisation test. *J. Invest. Dermatol.,* 1969, vol. 52 (3), 268-276
- Hen's Egg Test - Chorioallantoic Membrane. *J. O.,* 26 Décembre 1996